(19) Europäisches Patentamt
European Patent Office
Office européen des brevets

(11) Veröffentlichungsnummer: **0 315 003 A2**

(12) # EUROPÄISCHE PATENTANMELDUNG

(21) Anmeldenummer: 88117656.4

(22) Anmeldetag: 24.10.88

(51) Int. Cl.4: **C07H 7/04 , C07H 15/26 , A61K 31/70**

(30) Priorität: 31.10.87 DE 3736960

(43) Veröffentlichungstag der Anmeldung: 10.05.89 Patentblatt 89/19

(84) Benannte Vertragsstaaten: BE CH DE FR GB IT LI NL

(71) Anmelder: HOECHST AKTIENGESELLSCHAFT
Postfach 80 03 20
D-6230 Frankfurt am Main 80(DE)

(72) Erfinder: Kretzschmar, Gerhard, Dr.
Falkenstrasse 71
D-6232 Bad Soden am Taunus(DE)
Erfinder: Düwel, Dieter, Dr.
Frankfurter Strasse 39
D-6238 Hofheim am Taunus(DE)
Erfinder: Grabley, Susanne, Dr.
Hölderlinstrasse 7
D-6240 Königstein/Taunus(DE)
Erfinder: Hammann, Peter, Dr.
Mörikestrasse 6
D-6233 Kelkheim/Taunus(DE)
Erfinder: Voelskow, Hartmut, Dr.
Akazienstrasse 22
D-6234 Hattersheim am Main(DE)
Erfinder: Giani, Carlo, Dr.
Hedderichstrasse 69
D-6000 Frankfurt am Main 70(DE)
Erfinder: Seibert, Gerhard, Prof. Dr.
Gläserweg 21
D-6100 Darmstadt(DE)

(54) **Elaiophylinderivate, Verfahren zu deren Herstellung, diese enthaltende Mittel und Verwendung derselben.**

(57) Die Erfindung betrifft Elaiophylinderivate der Formel I,

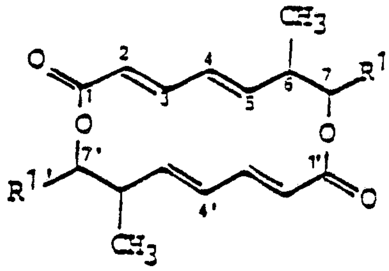

(I)

worin R' und R'′ gleich oder verschieden sind und einen Rest der Formel II und/oder III darstellen

EP 0 315 003 A2

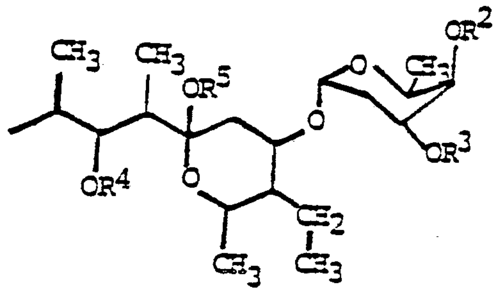

(II)

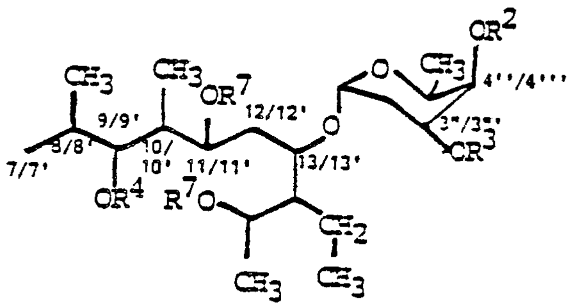

(III)

worin $R^2$, $R^3$, $R^4$, $R^5$ und $R^7$ die angegebenen Bedeutungen haben. Weiterhin betrifft die Erfindung ein Verfahren zur Herstellung dieser Elaiophylinderivate sowie die Verwendung derselben als Arzneimittel, insbesondere als anthelminthisch wirkende Arzneimittel.

## Elaiophylinderivate, Verfahren zu deren Herstellung, diese enthaltende Mittel und Verwendung derselben

Elaiophylin wurde vor über 25 Jahren zuerst von Arcamone et al. (giorn. Microbiol. 7, 207, 1959) isoliert und später von Arai unter dem Namen Azalomycin B beschrieben. Kaiser et al. (Helv. Chim. Acta, 64 (1981), 407) isolierten Elaiophylin zusammen mit Nigericin und den Niphitricinen A und B aus Kulturen eines Stammes von Streptomyces violaceoniger.

Es wurde bereits vorgeschlagen (P 37 21 722.4), Elaiophylin als Anthelminthikum einzusetzen, da es insbesondere eine gute Wirkung gegen Magen-Darm-Strongyliden und Lungenwürmer entfaltet, von denen vor allem Haus- und Nutztiere befallen werden.

Es wurde nun gefunden, daß durch chemische Derivatisierung des Elaiophylins die anthelminthische Wirkung noch gesteigert werden kann.

Einige Derivate des Elaiophylins sind bereits bekannt, jedoch liegt ihr pharmakologisches Wirkungsspektrum weitab von der hier beschriebenen anthelminthischen Wirkung; meist werden diese Derivate als Ulkustherapeutika eingesetzt.

Bekannt sind bereits einige Alkyl-, Alkenyl- und Alkinyl-Derivate sowie Phenyl- und Furylalkyl-Derivate in 11- und 11′-Position am Elaiophylin (Japanische Offenlegungsschrift 61-36295, YOKURA et al.). Ebenfalls bekannt sind die reduzierten und teilreduzierten Halbacetale des Elaiophylins, wobei der 6′-Ring am C11 bzw. am C11 und C11′ geöffnet ist sowie die Tetraacetylderivate (3″, 3‴, 4″, 4‴-Tetra-O-acetyl) des Elaiophylins (s. loc. cit. Kaiser et al.). Sowohl von den reduzierten Halbacetalen als auch von den Tetraacetylderivaten sind schon die Verbindungen bekannt, in denen die C-C-Doppelbindungen des Macrodiolidrings vollständig hydriert sind (s. loc. cit. Kaiser et al.).

Die Erfindung betrifft nun:

Elaiophylinderivate der Formel I,

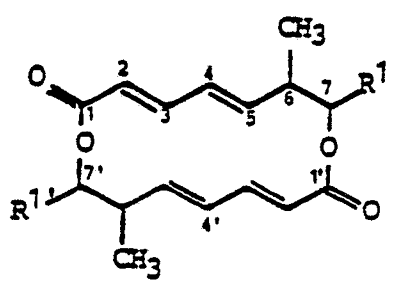

(I)

wobei die C-C-Doppelbindungen in dem Macrodiolidring der Verbindung der Formel (I) auch hydriert sein können und in der

$R^1$ ein Rest der Formel II oder III ist,

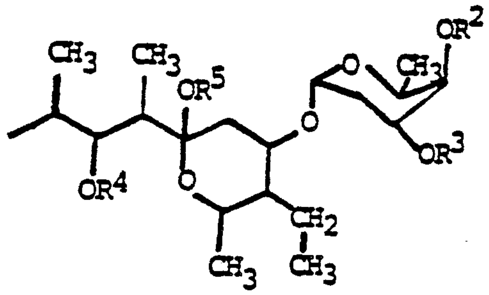

(II)

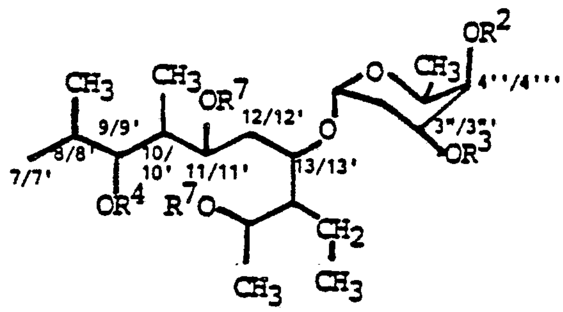

(III)

worin
$R^2$ und $R^3$ gleich oder verschieden sind und Wasserstoff oder einen Rest der Formel IV oder IV′

$$-\overset{\overset{\displaystyle O}{\|}}{C}-R^6 \qquad (IV)$$

$$-\overset{\overset{\displaystyle O}{\|}}{C}-(CH_2)_n-R^6 \qquad (IV')$$

bedeuten, in der
n 1 bis 3 und
$R^6$ $C_1$-$C_{15}$-Alkyl, $C_1$-$C_{15}$-Alkenyl, $C_2$-$C_{15}$-Alkinyl, $C_3$-$C_9$-Cycloalkyl, Aryl- oder Heteroaryl bedeutet, wobei die Aryl- und Heteroarylreste gegebenenfalls halogen-, nitro-, cyano-, hydroxy, $C_1$-$C_4$-alkyl- oder $C_1$-$C_4$-alkoxysubstituiert sind
und wobei für den Fall, daß $R^3$ Wasserstoff ist, $R^2$ auch Wasserstoff ist,
oder worin
$R^2$ und $R^3$ gleich sind und
$C_1$-$C_4$-Alkyl, Benzyl, Allyl, MEM, MOM, SEM, Trimethylsilyl, Triethylsilyl, Dimethylphenylsilyl, Dimethylcyclohexylsilyl oder Dimethyltertiärbutylsilyl bedeutet
oder
$R^2$ und $R^3$ Sulfonsäureester der Formel $SO_2R^{10}$ bedeuten, in der
$R^{10}$ $C_1$-$C_{10}$-Alkyl, Phenyl oder p-Toloyl bedeutet
oder
$R^2$ und $R^3$ einen Rest der Formel V oder V′ darstellen,

$$-\overset{\overset{\displaystyle X}{\|}}{C}-Z-R^{12} \qquad (V)$$

$$-\overset{\overset{\displaystyle X}{\|}}{C}-Z-(CH_2)_n-R^{12} \qquad (V')$$

in der
n 1 bis 3

X Sauerstoff oder Schwefel
$R^{12}$ $C_1$-$C_{15}$-Alkyl, $C_3$-$C_9$-Cycloalkyl, Aryl, Pyridyl, Pyrimidyl oder Pyrazinyl bedeutet, wobei die Aryl-, Pyridyl-, Pyrimidyl- oder Pyrazinylreste gegebenenfalls halogen-, nitro-, cyano- oder $C_1$-$C_4$-alkoxysubstituiert sind und
Z Sauerstoff, -N-H oder einen Rest $-N-R^{12}$ oder $-N-(CH_2)_n-R^{12}$ bedeutet, wobei n und $R^{12}$ die oben angegebenen Bedeutungen haben
oder worin
$R^2$ und $R^3$ zusammen einen Rest der Formel VI darstellen

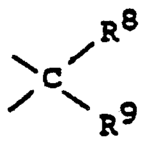

(VI)

in der
$R^8$ und $R^9$ gleich oder verschieden sind und Wasserstoff oder unverzweigtes $C_1$-$C_{10}$-Alykl bedeuten oder wobei
$R^8$ und $R^9$ eine Alkylenkette darstellt, die zusammen mit dem sie tragenden C-Atom einen 5-, 6- oder 7-gliedrigen Ring bildet
oder worin
$R^2$ und $R^3$ für den Fall, daß $R^1$ ein Rest der Formel II ist, gleich sind und einen Rest der Formel VIII darstellen,

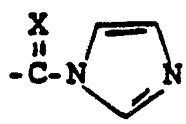

(VIII)

in der
X Sauerstoff oder Schwefel bedeutet
oder
$R^2$ und $R^3$ für den Fall, daß $R^1$ ein Rest der Formel II ist, zusammen einen Rest der Formel $>C=X$ darstellen, worin
X Sauerstoff oder Schwefel bedeutet
und worin
$R^4$ sofern $R^2$ und $R^3$ Wasserstoff sind, Wasserstoff bedeutet oder für den Fall, daß $R^2$ und $R^3$ nicht Wasserstoff bedeuten, Wasserstoff oder ein Rest der Formel IV oder IV′ ist,

$$-\overset{O}{\overset{\|}{C}}-R^6 \qquad (IV)$$

$$-\overset{O}{\overset{\|}{C}}-(CH_2)_n-R^6 \qquad (IV')$$

in der
n und $R^6$ die oben angegebenen Bedeutungen haben
und
$R^5$ Wasserstoff oder ein Rest der Formel $-(CH_2)_n-R^6$ ist,
wobei
n und $R^6$ die oben angegebenen Bedeutungen haben
und worin
$R^7$ Wasserstoff oder - sofern $R^2$, $R^3$ und $R^4$ nicht gleichzeitig Wasserstoff bedeuten - einen Rest der Formel IV oder IV′ - wie oben definiert -bildet
und in der

$R^{1'}$ die gleiche Bedeutung wie $R^1$ hat, wobei die beiden Reste $R^1$ und $R^{1'}$ sowohl gleich als auch - bis auf die Reste $R^4$ und $R^5$ und für den Fall, daß $R^2$ und $R^3$ zusammen einen Rest der Formel VI bilden - verschieden substituiert sein können, mit der Ausnahme, daß wenn $R^1$ ein Rest der Formel II ist, in der $R^2$ und $R^3$ ein Rest der Formel VIII oder in der $R^2$ und $R^3$ zusammen einen Rest der Formel $>C=X$ bilden, für $R^{1'}$ die Reste $R^2$ und $R^3$ im Rest der Formel II oder III nicht gleichzeitig Wasserstoff sind, ausgenommen Elaiophylin selbst, Elaiophylin mit hydriertem Macrodiolidring, sowie Verbindungen der Formel I, worin der Macrodiolidring hydriert oder nicht hydriert ist und in der $R^1$ und $R^{1'}$ gleich oder verschieden sind und einen Rest der Formel II und/oder III darstellen, worin $R^2$ und $R^3$ Wasserstoff oder Acetyl und $R^4$ und $R^5$ oder $R^7$ Wasserstoff bedeutet sowie ebenfalls ausgenommen die Verbindungen der Formel I, in der der Macrodiolidring nicht hydriert ist und worin $R^1$ und $R^{1'}$ gleichzeitig einen Rest der Formel II darstellen, worin $R^2$, $R^3$ und $R^4$ Wasserstoff bedeuten und worin
$R^5$ $C_1$-, $C_2$-, $C_4$-$C_8$, $C_{10}$- oder $C_{12}$-Alkyl, Phenyl-$C_1$-$C_3$-alkyl, para-Methoxy-phenyl-methyl, Cyclohexylmethyl, $C_4$-Alkenyl oder $C_3$-Alkinyl
bedeutet.

Insbesondere betrifft die Erfindung Elaiophylinderivate der Formel I, wobei die C-C-Doppelbindungen in dem Macrodiolidring der Verbindung der Formel I auch hydriert sein können und in der $R^1$ ein Rest der Formel II oder III ist, worin
$R^2$ und $R^3$ gleich oder verschieden sind und Wasserstoff oder einen Rest der Formel IV oder IV' bedeutet,
in der
n 1 bis 3 und
$R^6$ $C_1$-$C_5$-Alkyl, $C_2$-$C_5$-Alkenyl, $C_2$-$C_6$-Alkinyl, $C_3$-$C_6$-Cycloalkyl, Phenyl oder Furyl oder Thienyl bedeutet, wobei die Phenyl-, Furyl- und Thienylreste gegebenenfalls halogen-, nitro-, cyano-, hydroxy-, $C_1$-$C_4$-alkyl- oder $C_1$-$C_4$-alkoxysubstituiert sind
und wobei für den Fall, daß $R^3$ Wasserstoff ist, $R^2$ auch Wasserstoff ist,
oder worin
$R^2$ und $R^3$ gleich sind und MEM, MOM, SEM, Trimethylsilyl, Triethylsilyl, Dimethylphenylsilyl, Dimethylcyclohexylsilyl oder Dimethyltertiärbutylsilyl bedeutet
oder
$R^2$ und $R^3$ Sulfonsäureester der Formel $SO_2R^{10}$ bedeuten,
in der
$R^{10}$ $C_1$-$C_4$-Alkyl, Phenyl oder p-Toloyl bedeutet
oder
$R^2$ und $R^3$ einen Rest der Formel V oder V' darstellen,
in der
n 1,
X Sauerstoff,
$R^{12}$ $C_1$-$C_4$-Alkyl, $C_3$-$C_6$-Cycloalkyl oder Aryl bedeutet, wobei der Arylrest gegebenenfalls halogen-, nitro-, cyano- oder $C_1$-$C_4$-alkoxysubstituiert ist und
Z Sauerstoff oder -N-H bedeutet
oder worin
$R^2$ und $R^3$ zusammen einen Rest der Formel VI darstellen
in der
$R^8$ und $R^9$ gleich oder verschieden sind und Wasserstoff oder unverzweigtes $C_1$-$C_4$-Alykl bedeuten oder wobei
$R^8$ und $R^9$ eine Alkylenkette darstellt, die zusammen mit dem sie tragenden C-Atom einen 5- oder 6-gliedrigen Ring bildet
oder worin
$R^2$ und $R^3$ für den Fall, daß $R^1$ ein Rest der Formel II ist, gleich sind und einen Rest der Formel VIII darstellen,
in der
X Sauerstoff bedeutet
oder
$R^2$ und $R^3$ für den Fall, daß $R^1$ ein Rest der Formel II ist, zusammen einen Rest der Formel $>C=X$ darstellen,
worin
X Sauerstoff bedeutet
und worin
$R^4$ sofern $R^2$ und $R^3$ Wasserstoff sind, Wasserstoff bedeutet oder für den Fall, daß $R^2$ und $R^3$ nicht

Wasserstoff bedeuten. Wasserstoff oder ein Rest der Formel IV oder IV′ ist,
in der
n 1 bis 3 ist und $R^6$ die oben angegebenen Bedeutungen hat,
und
$R^5$ Wasserstoff oder ein Rest der Formel $-(CH_2)_n-R^6$ ist,
wobei
n 1 bis 3 ist und $R^6$ die oben angegebenen Bedeutungen hat
und worin
$R^7$ Wasserstoff oder - sofern $R^2$, $R^3$ und $R^4$ nicht gleichzeitig Wasserstoff bedeuten - einen Rest der Formel IV oder IV′ - wie oben definiert - bildet
und in der
$R^{1'}$ die gleiche Bedeutung wie $R^1$ hat, wobei die beiden Reste $R^1$ und $R^{1'}$ sowohl gleich als auch - bis auf die Reste $R^4$ und $R^5$ und für den Fall, daß $R^2$ und $R^3$ zusammen einen Rest der Formel VI bilden - verschieden substituiert sein können, mit der Ausnahme, daß wenn $R^1$ ein Rest der Formel II ist, in der $R^2$ und $R^3$ ein Rest der Formel VIII oder in der $R^2$ und $R^3$ zusammen einen Rest der Formel $>C=X$ bilden, für $R^{1'}$ die Reste $R^2$ und $R^3$ im Rest der Formel II oder III nicht gleichzeitig Wasserstoff sind, ausgenommen Elaiophylin selbst, Elaiophylin mit hydriertem Macrodiolidring, sowie Verbindungen der Formel I, worin der Macrodiolidring hydriert oder nicht hydriert ist und in der $R^1$ und $R^{1'}$ gleich oder verschieden sind und einen Rest der Formel II und/oder III darstellen, worin $R^2$ und $R^3$ Wasserstoff oder Acetyl und $R^4$ und $R^5$ oder $R^7$ Wasserstoff bedeutet sowie ebenfalls ausgenommen die Verbindungen der Formel I, in der der Macrodiolidring nicht hydriert ist und worin $R^1$ und $R^{1'}$ gleichzeitig einen Rest der Formel II darstellen, worin $R^2$, $R^3$ und $R^4$ Wasserstoff bedeuten und worin
$R^5$ $C_1$-, $C_2$-, $C_4$-$C_5$-Alkyl, Phenyl-$C_1$-$C_3$-Alkyl, para-Methoxy-phenyl-methyl, Cyclohexylmethyl, $C_4$-Alkenyl oder $C_3$-Alkinyl
bedeutet.

Besonders bevorzugt sind Elaiophylinderivate der Formel I, wobei die C-C-Doppelbindungen in dem Macrodiolidring der Verbindung der Formel I auch hydriert sein können und in der $R^1$ ein Rest der Formel II oder III ist,
worin
$R^2$ und $R^3$ gleich oder verschieden sind und Wasserstoff oder einen Rest der Formel IV oder IV′ bedeuten,
in der
n 1
$R^6$ $C_1$-$C_5$-Alkyl, Cyclohexyl, Phenyl, Furyl oder Thienyl bedeutet, wobei die Phenyl-, Furyl-, und Thienylreste gegebenenfalls mit Fluor, Chlor oder Brom substituiert sind
und wobei für den Fall, daß $R^3$ Wasserstoff ist, $R^2$ auch Wasserstoff ist,
oder worin
$R^2$ und $R^3$ gleich sind und MEM, MOM, SEM, Trimethylsilyl, Triethylsilyl, Dimethylphenylsilyl, Dimethylcyclohexylsilyl oder Dimethyltertiärbutylsilyl bedeutet
oder
$R^2$ und $R^3$ Sulfonsäureester der Formel $SO_2R^{10}$ bedeuten, in der
$R^{10}$ Methyl, Phenyl oder p-Toloyl bedeutet
oder
$R^2$ und $R^3$ einen Rest der Formel V oder V′ darstellen,
in der
n 1
X Sauerstoff
$R^{12}$ Cyclohexyl oder Aryl bedeutet, wobei der Arylrest gegebenenfalls halogen-, nitro-, cyano- oder $C_1$-$C_4$-alkoxysubstituiert ist und
Z Sauerstoff oder -N-H bedeutet,
oder worin
$R^2$ und $R^3$ zusammen einen Rest der Formel VI darstellen
in der
$R^8$ und $R^9$ gleich oder verschieden sind und Wasserstoff oder unverzweigtes $C_1$-$C_4$-Alykl bedeuten oder wobei
$R^8$ und $R^9$ eine Alkylenkette darstellt, die zusammen mit dem sie tragenden C-Atom einen 5- oder 6-gliedrigen Ring bildet
oder worin
$R^2$ und $R^3$ für den Fall, daß $R^1$ ein Rest der Formel II ist, gleich sind und einen Rest der Formel VIII

darstellen,
in der
X Sauerstoff bedeutet
oder
$R^2$ und $R^3$ für den Fall, daß $R^1$ ein Rest der Formel II ist, zusammen einen Rest der Formel $>C=X$ darstellen,
worin
X Sauerstoff bedeutet
und worin
$R^4$ sofern $R^2$ und $R^3$ Wasserstoff sind, Wasserstoff bedeutet oder für den Fall, daß $R^2$ und $R^3$ nicht Wasserstoff bedeuten, Wasserstoff oder ein Rest der Formel IV oder IV′ ist,
in der
n 1 ist und $R^6$ die oben angegebenen Bedeutungen hat
und
$R^5$ Wasserstoff oder ein Rest der Formel $-(CH_2)_n-R^6$ ist,
wobei
n 1 ist und $R^6$ die oben angegebenen Bedeutungen hat
und worin
$R^7$ Wasserstoff oder - sofern $R^2$, $R^3$ und $R^4$ nicht gleichzeitig Wasserstoff bedeuten - einen Rest der Formel IV oder IV′ - wie oben definiert -bildet
und in der
$R^{1'}$ die gleiche Bedeutung wie $R^1$ hat, wobei die beiden Reste $R^1$ und $R^{1'}$ sowohl gleich als auch - bis auf die Reste $R^4$ und $R^5$ und für den Fall, daß $R^2$ und $R^3$ zusammen einen Rest der Formel VI bilden - verschieden substituiert sein können, mit der Ausnahme, daß wenn $R^1$ ein Rest der Formel II ist, in der $R^2$ und $R^3$ ein Rest der Formel VIII oder in der $R^2$ und $R^3$ zusammen einen Rest der Formel $C=X$ bilden, für $R^{1'}$ die Reste $R^2$ und $R^3$ im Rest der Formel II oder III nicht gleichzeitig Wasserstoff sind,
ausgenommen Elaiophylin selbst, Elaiophylin mit hydriertem Macrodiolidring, sowie Verbindungen der Formel I, worin der Macrodiolidring hydriert oder nicht hydriert ist und in der $R^1$ und $R^{1'}$ gleich oder verschieden sind und einen Rest der Formel II und/oder III darstellen, worin $R^2$ und $R^3$ Wasserstoff oder Acetyl und $R^4$ und $R^5$ oder $R^7$ Wasserstoff bedeutet sowie ebenfalls ausgenommen die Verbindungen der Formel I, in der der Macrodiolidring nicht hydriert ist und worin $R^1$ und $R^{1'}$ gleichzeitig einen Rest der Formel II darstellen, worin $R^2$, $R^3$ und $R^4$ Wasserstoff bedeuten und worin
$R^5$ $C_1$-$C_2$-, $C_4$-$C_5$-Alkyl, Phenylmethyl oder Cyclohexylmethyl, bedeutet.

Weiterhin betrifft die Erfindung Elaiophylinderivate der Formel I, gemäß Anspruch 1, jedoch einschließlich Elaiophylin mit hydriertem Macrodiolidring, sowie Verbindungen der Formel I, worin der Macrodiolidring hydriert oder nicht hydriert ist und in der $R^1$ und $R^{1'}$ gleich oder verschieden sind und einen Rest der Formel II und/oder III darstellen, worin $R^2$ und $R^3$ Wasserstoff oder Acetyl und $R^4$ und $R^5$ oder $R^7$ Wasserstoff bedeutet sowie ebenfalls eingeschlossen die Verbindungen der Formel I, in der der Macrodiolidring nicht hydriert ist und worin $R^1$ und $R^{1'}$ gleichzeitig einen Rest der Formel II darstellen, worin $R^2$, $R^3$ und $R^4$ Wasserstoff bedeuten und worin
$R^5$ $C_1$-, $C_2$-, $C_4$-$C_8$, $C_{10}$- oder $C_{12}$-Alkyl, Phenyl-$C_1$-$C_3$-alkyl, para-Methoxy-phenyl-methyl, Cyclohexylmethyl, $C_4$-Alkenyl oder $C_3$-Alkinyl.
bedeutet,
zur Anwendung als Anthelminthikum.

Bevorzugt sind Elaiophylinderivate der Formel I, gemäß Anspruch 2, jedoch einschließlich Elaiophylin mit hydriertem Macrodiolidring, sowie Verbindungen der Formel I, worin der Macrodiolidring hydriert oder nicht hydriert ist und in der $R^1$ und $R^{1'}$ gleich oder verschieden sind und einen Rest der Formel II und/oder III darstellen, worin $R^2$ und $R^3$ Wasserstoff oder Acetyl und $R^4$ und $R^5$ oder $R^7$ Wasserstoff bedeutet sowie ebenfalls eingeschlossen die Verbindungen der Formel I, in der der Macrodiolidring nicht hydriert ist und worin $R^1$ und $R^{1'}$ gleichzeitig einen Rest der Formel II darstellen, worin $R^2$, $R^3$ und $R^4$ Wasserstoff bedeuten und worin
$R^5$ $C_1$-, $C_2$-, $C_4$-$C_5$-Alkyl, Phenyl-$C_1$-$C_3$-Alkyl, para-Methoxy-phenyl-methyl, Cyclohexylmethyl, $C_4$-Alkenyl oder $C_3$-Alkinyl
bedeutet,
zur Anwendung als Anthelminthikum.

Besonders bevorzugt sind Elaiophylinderivate der Formel I, gemäß Anspruch 3, jedoch einschließlich Elaiophylin mit hydriertem Macrodiolidring, sowie Verbindungen der Formel I, worin der Macrodiolidring hydriert oder nicht hydriert ist und in der $R^1$ und $R^{1'}$ gleich oder verschieden sind und einen Rest der

Formel II und/oder III darstellen, worin $R^2$ und $R^3$ Wasserstoff oder Acetyl und $R^4$ und $R^5$ oder $R^7$ Wasserstoff bedeutet sowie ebenfalls eingeschlossen die Verbindungen der Formel I, in der der Macrodiolidring nicht hydriert ist und worin $R^1$ und $R^{1'}$ gleichzeitig einen Rest der Formel II darstellen, worin $R^2$, $R^3$ und $R^4$ Wasserstoff bedeuten und worin $R^5$ $C_1$-$C_2$-, $C_4$-$C_5$-Alkyl, Phenylmethyl oder Cyclohexylmethyl, bedeutet, zur Anwendung Als Anthelminthikum.

Ebenso betrifft die Erfindung ein Verfahren zur Herstellung von Elaiophylinderivaten der Formel I, das dadurch gekennzeichnet ist, daß man

a) Elaiophylin (Formel I: $R^1 = R^{1'} = H$, $R^2 = R^3 = R^4 = R^5 = H$) oder eine Verbindung der Formel I, in der $R^1$ und $R^{1'}$ gleich oder verschieden sind und einen Rest der Formel II und/oder III darstellen, worin $R^2$, $R^3$ und $R^4$ die oben angegebenen Bedeutung haben und $R^5$ oder $R^7$ bis auf Wasserstoff die oben angegebenen Bedeutungen haben, umsetzt mit einer Verbindung der Formel IX oder IX$'$

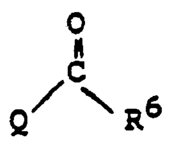
(IX)
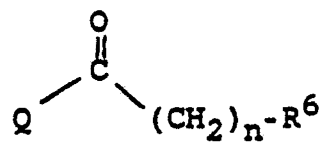
(IX')

in der n und $R^6$ die oben zu Formel IV oder IV$'$ angegebenen Bedeutungen haben und Q Chlorid, Bromid, ein Imidazolid oder ein Säureanhydrid bedeutet, wobei man Verbindungen der Formel I erhält, in der $R^1$ und $R^{1'}$ gleich oder verschieden sind und einen Rest der Formel II und/oder III darstellen, in der $R^2$ und $R^3$ gleich oder verschieden sind und einen Rest der Formel IV oder IV$'$, wie oben definiert, darstellen, und in der $R^4$ und $R^5$ oder $R^7$ gegenüber der Ausgangsverbindung unverändert bleiben, oder daß man

b) ein Elaiophylin-Derivat der Formel I in der $R^1$ und $R^{1'}$ gleich oder verschieden sind und einen Rest der Formel II und/oder III darstellen, worin $R^2$, $R^3$ und $R^4$ die oben angegebenen Bedeutungen haben und $R^5$ oder $R^7$ bis auf Wasserstoff die oben angegebenen Bedeutungen haben, umsetzt mit einer Verbindung, ausgewählt aus:
$C_1$-$C_4$-Alkyl-, Benzyl-, Allylchlorid, -bromid, -jodid oder MEM-, MOM-, SEM-chlorid, Trimethylsilyl, Triethylsilyl-, Dimethylphenylsilyl-, Dimethylcyclohexylsilyl-, Dimethyltertiärbutylsilyl-chlorid Sulfonsäurehalogeniden der Formel Y-$SO_2R^{10}$, wobei Y Chlor, Brom oder Jod bedeutet und $R^{10}$ die oben angegebenen Bedeutungen hat, Isocyanaten der Formel $O = C = N\text{-}R^{12}$ oder
$O = C = N\text{-}(CH_2)_n\text{-}R^{12}$, Isothiocyanaten der Formel $S = C = N\text{-}R^{12}$ oder
$S = C = N\text{-}(CH_2)_n\text{-}R^{12}$, Carbaminsäurehalogeniden der Formel $Y\text{-}CO\text{-}N\text{-}(R^{12})_2$ oder
$Y\text{-}CO\text{-}N\text{-}[(CH_2)_n\text{-}R^{12}]_2$, Thiocarbaminsäurehalogeniden der Formel $Y\text{-}CS\text{-}N\text{-}(R^{12})_2$ oder
$Y\text{-}CS\text{-}N\text{-}[(CH_2)_n\text{-}R^{12}]_2$,
wobei n und $R^{12}$ die oben zu Formel V oder V$'$ angegebenen Bedeutungen haben und Y Chlor oder Brom bedeutet, wobei man Verbindungen der Formel I erhält, in der $R^1$ und $R^{1'}$ gleich oder verschieden sind und einen Rest der Formel II und/oder III darstellen, in der $R^2$ und $R^3$ gleich sind und einen
$C_1$-$C_4$-Alkyl-, Benzyl-, Allyl-, MEM-, MOM-, SEM-, Trimethylsilyl-, Triethylsilyl-, Dimethylphenylsilyl-, Dimethylcyclohexylsilyl- oder Dimethyltertiärbutylsilyl-Rest darstellen oder einen Sulfonsäureester der Formel $SO_2R^{10}$ oder einen Rest der Formel V oder V$'$ bedeuten, wobei $R^{10}$ und die Formeln V und V$'$ die oben angegebenen Bedeutungen haben und in der $R^4$ und $R^5$ oder $R^7$ gegenüber der Ausgangsverbindung unverändert sind, oder daß man

c) Elaiophylin (Formel I: $R^1 = R^{1'} = H$, $R^2 = R^3 = R^4 = R^5 = H$) umsetzt mit einem Aldehyd/Keton der Formel X

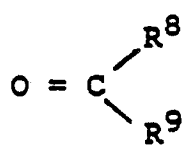
(X)

oder mit einem Ketal der Formel XI

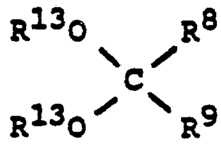

(XI)

worin $R^{'3}$ Methyl oder Ethyl bedeutet und $R^8$ und $R^9$ die oben angegebenen Bedeutungen haben, wobei man Verbindungen der Formel I erhält, in der $R^1$ und $R^{1'}$ gleich sind und einen Rest der Formel II darstellen, in der $R^2$ und $R^3$ zusammen einen Rest der Formel VI, wie oben definiert, darstellen oder daß man

d) Elaiophylin (Formel: $R^1 = R^{1'} = II$, $R^2 = R^3 = R^4 = R^5 = H$) oder eine Verbindung der Formel I, in der $R^1$ und $R^{1'}$ gleich oder verschieden sind und einen Rest der Formel II darstellen, worin $R^2$, $R^3$ und $R^4$ die oben angegebenen Bedeutungen haben und $R^5$ bis auf Wasserstoff die oben angegebenen Bedeutungen hat, gegebenenfalls in Gegenwart einer Base umsetzt mit Carbonyldiimidazol oder Thiocarbonyldiimidazol, wobei man Verbindungen der Formel I erhält, in der $R^1$ und $R^{1'}$ gleich oder verschieden sind und einen Rest der Formel II darstellen, in der $R^2$ und $R^3$ gleich sind und einen Rest der Formel VIII, wie oben definiert, darstellen oder in der $R^2$ und $R^3$ zusammen einen Rest der Formel $>C=X$, wie oben definiert, darstellen und in der $R^4$ und $R^5$ gegenüber der Ausgangsverbindung unverändert sind, oder daß man

e) eine Verbindung der Formel I, in der $R^1$ und $R^{1'}$ gleich oder verschieden sind und einen Rest der Formel II und/oder III darstellen, in der $R^2$ und $R^3$ bis auf Wasserstoff die oben angegebenen Bedeutungen haben und in der $R^4$ Wasserstoff bedeutet und $R^5$ oder $R^7$ bis auf Wasserstoff die oben angegebenen Bedeutungen hat, gemäß Verfahrensvariante a) mit einer Verbindung der Formel IX oder IX', wie unter a) definiert, umsetzt, wobei man Verbindungen der Formel I erhält, in der $R^1$ und $R^{1'}$ gleich oder verschieden sind und einen Rest der Formel II und/oder III darstellen, in der $R^2$ und $R^3$ bis auf Wasserstoff die oben angegebenen Bedeutungen haben und $R^4$ ein Rest der Formel IV oder IV', wie oben definiert, bedeutet, wobei $R^4$ in $R^{1'}$ identisch ist mit $R^4$ in $R^1$ und in der $R^5$ oder $R^7$ gegenüber der Ausgangsverbindung unverändert ist, oder daß man

f) Elaiophylin oder eine Verbindung der Formel I, in der $R^1$ und $R^{1'}$ gleich oder verschieden sind und einen Rest der Formel II darstellen, in der $R^2$, $R^3$ und $R^4$ die oben angegebenen Bedeutungen haben und in der $R^5$ Wasserstoff bedeutet, bevorzugt in Gegenwart einer Lewis-Säure mit einem Alkohol der Formel $HO-(CH_2)_n-R^6$ oder in der n und $R^6$ die oben angegebenen Bedeutungen haben, umsetzt, wobei man Verbindungen der Formel I erhält, in der $R^1$ und $R^{1'}$ gleich oder verschieden sind und einen Rest der Formel II darstellen, in der $R^2$ und $R^3$ und $R^4$ die oben angegebenen Bedeutungen haben und $R^5$ in den Resten $R^1$ und $R^{1'}$ identisch ist und einen Rest der Formel $-(CH_2)_n-R^6$ oder $-R^6$, wie oben definiert, darstellt, oder daß man

g) Elaiophylin (Formel I: $R^1 = R^{1'} = II$, $R^2 = R^3 = R^4 = R^5 = H$) oder eine Verbindung der Formel I, in der $R^1$ und $R^{1'}$ gleich oder verschieden sind und einen Rest der Formel II darstellen, worin $R^2$, $R^3$ und $R^4$ die oben angegebenen Bedeutungen haben und $R^5$ Wasserstoff ist, wobei für $R^2$ und $R^3$ die Substituenten der Formel VIII sowie die Carbonyl- und Thiocarbonylreste ausgeschlossen sind, reduziert, wobei man eine Verbindung der Formel I erhält, in der $R^1$ und/oder $R^{1'}$ ein Rest der Formel III ist, worin $R^2$, $R^3$ und $R^4$ die oben angegebenen Bedeutungen haben und $R^7$ Wasserstoff bedeutet, oder daß man

h) eine Verbindung der Formel I, in der $R^1$ und/oder $R^{1'}$ ein Rest der Formel III ist, worin $R^2$, $R^3$ und $R^4$ die oben angegebenen Bedeutungen haben und $R^7$ Wasserstoff bedeutet, gemäß Verfahrensvariante a) mit einer Verbindung der Formel IX oder IX', wie in Verfahrensvariante a) definiert, zu einer Verbindung der Formel I umsetzt, in der $R^1$ und/oder $R^{1'}$ ein Rest der Formel III ist, worin $R^2$, $R^3$ und $R^4$ die oben angegebenen Bedeutungen haben oder, sofern vor der Umsetzung $R^2$ und/oder $R^3$ und/oder $R^4$ Wasserstoff bedeuteten, $R^2$ und/oder $R^3$ und/oder $R^4$ nach der Umsetzung die Bedeutung von $R^7$ haben und in der $R^7$ ein Rest der Formel IV oder IV', wie oben definiert, ist, oder daß man

i) eine Verbindung der Formel I, in der $R^1$ und/oder $R^{1'}$ ein Rest der Formel II und/oder III ist, worin $R^2$, $R^3$, $R^4$ und $R^5$ oder $R^7$ die oben angegebenen Bedeutungen haben, hydriert, wobei man eine hydrierte Verbindung der Formel I erhält, in der $R^1$ und/oder $R^{1'}$ ein Rest der Formel II und/oder III ist, worin $R^2$, $R^3$, $R^4$ und $R^5$ oder $R^7$ die oben angegebenen Bedeutungen haben.

Unter Aryl werden aromatische Kohlenwasserstoffe, insbesondere Phenyl und Naphthyl verstanden und unter Heteroaryl, heteroaromatische Kohlenwasserstoffe, insbesondere Thiophen und Furan, aber auch Pyridin, Pyrimidin und Pyrazin.

Unter Halogenen werden Fluor, Chlor, Brom und Jod verstanden, unter MEM Methoxyethoxymethyl.

unter MOM Methoxymethyl und unter SEM β-Trimethylsilylethoxymethyl.

Der Macrodiolidring des Elaiophilyins kann - unabhängig von der Art der Substituenten $R^1$ und $R^{1'}$ - hydriert oder nicht hydriert sein.

Die Substituenten $R^1$ und $R^{1'}$ können sowohl gleich als auch verschieden sein, d.h. es ist sowohl möglich, daß $R^1$ und $R^{1'}$ einen Rest der Formel II oder III darstellen als auch daß $R^1$ einen Rest der Formel II darstellt und $R^{1'}$ einen Rest der Formel III darstellt (die Umkehr: $R^1$ = ein Rest der Formel III und $R^{1'}$ = ein Rest der Formel II ist auf Grund der Symmetrie des Moleküls identisch mit $R^1$ = II und $R^{1'}$ = III). Ebenso ist es - bis auf wenige Ausnahmen -möglich, in den Resten $R^1$ und $R^{1'}$ unterschiedliche Reste $R^2$, $R^3$, $R^4$, $R^5$ bzw. $R^7$ einzubauen. Dies ist unabhängig davon, ob die Reste $R^1$ und $R^{1'}$ nun beide einen Rest der Formel II bzw. III darstellen, oder verschieden sind, also $R^1$ einen Rest der Formel II und $R^{1'}$ einen Rest der Formel III darstellen. Stellen $R^1$ und $R^{1'}$ jedoch einen Rest der Formel II dar, so ist $R^5$ in beiden Substituenten $R^1$ und $R^{1'}$ gleich.

Der Substituent $R^4$ ist in den Resten $R^1$ und $R^{1'}$ immer identisch und kann nur dann von Wasserstoff verschieden sein, wenn auch die Substituenten $R^2$ und $R^3$ von Wasserstoff verschieden sind. Ebenso kann $R^2$ nur dann von Wasserstoff verschieden sein, wenn auch $R^3$ von Wasserstoff verschieden ist.

Im folgenden werden die Verfahren a) bis h), die es ermöglichen die unterschiedlich substituierten Elaiophilyinderivate herzustellen, näher beschrieben.

Mit Hilfe der Verfahrensvarianten a) können die Hydroxylgruppen in 3″- bzw. 3‴-Stellung und 4″- bzw. 4‴-Stellung des Elaiophilyins verestert werden. Da die Reaktionsgeschwindigkeit der Veresterung in 3″- bzw. 3‴-Position - also für die $R^3$-Derivate - größer ist, als die Veresterung in 4″- bzw. 4‴-Position, eröffnet sich die Möglichkeit für $R^2$ und $R^3$ sowohl identische, also auch unterschiedliche Substituenten vorzusehen. Ebenso ist es auf diese Weise möglich, in den Substituenten $R^1$ und $R^{1'}$ unterschiedliche Reste $R^2$ und/oder $R^3$ einzubauen, Beispielsweise läßt sich Elaiophylin - oder dessen Homologes mit hydriertem Macrodiolidring - mit einem Reagenz A monoacetylieren (z.B. in 3″-Position) und anschließend - mit einem Reagenz B - in ein 3‴, 4″,4‴-Tri-O-acetyl(B)-3″-O-acetyl(A)-Derivat überführen. In ähnlicher Weise ist es durchaus möglich, vier unterschiedliche Substituenten in 3″-, 3‴-, 4″- und 4‴-Position in das Molekül einzuführen.

Bei höheren Temperaturen und/oder genügend langen Reaktionszeiten wird auch die OH-Gruppe in 9- bzw. 9′-Position verestert. Dies bedeutet aber auch, daß eine Veresterung in 9- bzw. 9′-Position nur nach vorheriger Veresterung der OH-Gruppen in 3″/3‴- und 4″/4‴-Position möglich ist. Auf diese Weise können also beispielsweise zunächst die OH-Gruppen in 3″/3‴- und 4″4‴-Position verestert werden und anschließend, gegebenenfalls nach Isolierung und Reinigung des Produktes, kann dann in einer zweiten Reaktion (Verfahrensvariante e) entsprechend Verfahrensvariante a die OH-Gruppe in 9-und/oder 9′-Stellung verestert werden.

Bei der Verfahrensvarianten a) geht man am besten so vor, daß man Elaiophylin ($R^1$ = $R^{1'}$ = II; $R^2$, $R^3$, $R^4$, $R^5$ = H) oder eine Verbindung der Formel I, in der $R^1$ und $R^{1'}$ gleich oder verschieden sind und einen Rest der Formel II und/oder III darstellen, worin $R^2$, $R^3$ und $R^4$ die oben angegebenen Bedeutungen haben und $R^5$ oder $R^7$ bis auf Wasserstoff die oben angegebenen Bedeutungen haben, in äquimolaren Mengen oder in bis zu einem 50-fachen Überschuß, gegebenenfalls in einem inerten, aprotischen Lösungsmittel wie Chloroform, Methylenchlorid, Tetrahydrofuran (THF), Essigester oder Dioxan, mit einer Verbindung der Formel IX oder IX′ bis zur Beendigung der Reaktion umsetzt, gegebenenfalls in Gegenwart einer Base, vorzugsweise Pyridin.

Die Reaktionstemperaturen liegen dabei zwischen -70 °C und +100 °C, vorzugsweise bei Verwendung eines Lösungsmittels zwischen dem Festpunkt und dem Siedepunkt des Lösungsmittels, insbesondere zwischen -70 °C und +40 °C. Die Reaktionszeiten betragen 1 bis 180 Stunden, bevorzugt 1 bis 48 Stunden, besonders bevorzugt 1 bis 8 Stunden. Die Beendiung der Reaktion kann beispielsweise mittels Dünnschichtchromatographie bestimmt werden (DC-Kontrolle).

Das als Ausgangssubstanz benötigte Elaiophylin kann beispielsweise nach dem in der deutschen Patentanmeldung P3721722.4 beschriebenen Verfahren hergestellt werden. Dabei fällt Elaiophilyin als Fermentationsprodukt von Kulturen der Stämme Streptomyces violaceoniger DSM 4137 bzw. Streptomyces parvulus DSM 3816 an.

Die Ausgangsverbindungen für die Verfahrensvariante a), die Verbindungen der Formel IX und/oder IX′ sind, sofern nicht käuflich, auf einfache Weise nach literaturbekannten Verfahren herstellbar. Beispielsweise erhält man die Säurechloride durch Umsetzung der entsprechenden Carbonsäure mit Thionylchlorid, $PCl_3$ oder $PCl_5$. Solche Verfahren sind beispielsweise beschrieben in Gattermann/Wieland, "Die Praxis des Organischen Chemikers", 43. Auflage, Walter der Gruyter, Berlin, New York 1982, S. 303 ff.

Bei der Verfahrensvarianten b) geht man am besten so vor, daß man das Elaiophylin oder ein Elaiophylin-Derivat in äquimolaren Mengen oder in einem bis zu 50-fachen Überschuß umsetzt; mit einer

Verbindung, die ausgewählt wird aus: $C_1$-$C_4$-Alkyl-, Benzyl-, Allylchlorid, -bromid, -jodid oder MEM-, MOM-, SEM-Chlorid, Trimethylsilyl-, Triethylsilyl-, Dimethylphenylsilyl-, Dimethylcyclohexylsilyl-, Dimethyltert.-butylsilyl-chlorid, Sulfonsäurehalogeniden der Formel $Y\text{-}SO_2R'^{0}$, Isocyanaten der Formel $O=C=N\text{-}R'^{2}$ oder $O=C=N\text{-}(CH_2)_n\text{-}R'^{2}$, Isothiocyanaten der Formel $S=C=N\text{-}R'^{2}$ oder $S=C=N\text{-}(CH_2)_n\text{-}R'^{2}$, Cabaminsäurehalogeniden der Formel $Y\text{-}CO\text{-}N\text{-}(R'^{2})_2$ oder $Y\text{-}CO\text{-}N\text{-}[(CH_2)_n\text{-}R'^{2}]_2$, Thiocarbaminsäurehalogeniden der Formel $Y\text{-}CS\text{-}N\text{-}(R'^{2})_2$ oder $Y\text{-}CS\text{-}N\text{-}[(CH_2)_n\text{-}R'^{2}]_2$.

Gegebenenfalls kann auch diese Umsetzung unter Basenzusatz erfolgen. Als Basen kommen beispielsweise Triethylamin, Pyridin oder Lutidin in Frage. Eine Variante des Verfahrens b) besteht darin, daß man in einem geeigneten, vorzugsweise inerten Lösungsmittel wie Chloroform, Methylenchlorid, THF, Essigester oder Dioxan arbeitet. Auch hier kann der Überschuß der oben aufgeführten Verbindungen bis zur 50-fachen Menge betragen.

Die Reaktionstemperaturen liegen dabei zwischen -70 °C und +100 °C, vorzugsweise bei Verwendung eines Lösungsmittels zwischen dem Festpunkt und dem Siedepunkt des Lösungsmittels, insbesondere zwischen -70 °C und -40 °C. Die Reaktionszeiten betragen 1 bis 180 Stunden, bevorzugt 1 bis 48 Stunden, besonders bevorzugt 1 bis 8 Stunden. Die Beendigung die Reaktion kann beispielsweise mittels DC-Kontrolle bestimmt werden.

Die Ausgangsverbindungen für die Verfahrensvarianten b) sind, sofern nicht käuflich, auf einfache Weise nach literaturbekannten Verfahren herstellbar. Beispielsweise erhält man die Sulfonsäurehalogenide der Formel $Y\text{-}SO_2R^{10}$ durch radikalische Umsetzung von Alkanen mit Chlor und $SO_2$ oder durch Halogenierung von Aromaten mit Halogensulfonsäure $Y\text{-}SO_3H$. Die Isocyanate, Isothiocyanate, Carbaminsäurehalogenide und Thiocarbaminsäurehalogenide erhält man nach literaturbekannten Verfahren wie z.B. in Houben-Weyl, 4. Auflage, Georg Thieme Verlag Stuttgart (1983), Band E4 beschrieben.

Mit Hilfe der Verfahrensvarinaten c) können die Hydroxylgruppen in 3″-3‴-Stellung und 4″-4‴-Stellung des Elaiophylins mit Resten $R^2$ und $R^3$, die zusammen einen Rest der Formel VI darstellen, acetalisiert werden.

Bei der Verfahrensvarianten c) geht man am besten so vor, daß man Elaiophilyin im Überschuß mit einer Verbindung der Formel X oder XI bis zur Beendigung der Reaktion umsetzt, gegebenenfalls in Gegenwart einer Lewis-Säure wie $ZnCl_2$, $ZnBr_2$, $FeCl_3$, $CuSO_4$ oder $CuCl_2$. Eine Variante des Verfahrens besteht darin, daß man in einem geeigneten aprotischen Lösungsmittel wie Chloroform, Methylenchlorid, THF, Essigester oder Dioxan arbeitet. Auch hier kann ein Überschuß an X oder XI angewandt werden.

Die Reaktionstemperaturen liegen dabei zwischen -20 °C und +100 °C, vorzugsweise bei Verwendung eines Lösungsmittels zwischen Raumtemperatur und dem Siedepunkt des Lösungsmittels, insbesondere zwischen 20 °C und 100 °C. Die Reaktionszeiten betragen 1 bis 50 Stunden, bevorzugt 1 bis 12 Stunden. Die Beendigung der Reaktion kann beispielsweise mittels DC-Kontrolle bestimmt werden.

Die Ausgangsverbindungen für die Verfahrensvariante c), die Verbindungen der Formel X und/oder XI sind, sofern nicht käuflich, auf einfache Weise nach literaturbekannten Verfahren herstellbar. Beispielsweise erhält man die Aldehyde der Formel X durch Oxidation von primären Alkoholen oder durch Reduktion von Carbonylderivaten, beispielsweise mit komplexen Hydriden oder durch Reduktion von Carbonsäurechloriden (weitere Beispiele zur Synthese von Aldehyden, Ketonen und Acetalen bzw. Ketalen finden sich in Organikum, Organisch Chemisches Praktikum, 15. Auflage, VEB Deutscher Verlag der Wissenschaften, Berlin 1976, Methodenregister, Acetale, Ketale, Aldeyhde, Ketone, S. 819 ff.).

Mit Hilfe der Verfahrensvariante d) lassen sich die Hydroxylgruppen in 3″-3‴-Stellung und 4″-4‴-Stellung des Elaiophylins in die $OR^2$-$OR^3$-Derivate überführen, in denen $R^2$ und/oder $R^3$ ein Carbonylimidazolid, Thiocarbonylimidazolid oder zusammen einen Carbonyl- oder Thiocarbonylrest darstellen. Die Reaktion läßt sich so steuern, daß beispielsweise die Rest $R^1$ und $R^{1'}$ identisch sind und die Substituten $R^2$ und $R^3$ ebenfalls identisch sind und Carbonyl- oder Thiocarbonylimidazol bedeuten. Ebenso läßt sich die Reaktion aber auch so steuern, daß beispielsweise in dem Rest $R^1$ die Substituenten $R^2$ und $R^3$ einen Carbonyl- oder Thiocarbonylrest darstellen und in dem Rest $R^{1'}$ die Substituenten $R^2$ und $R^3$ identisch sind und Carbonyl- oder Thiocarbonylimidazol bedeuten. Beeinflussen läßt sich diese Reaktion beispielsweise durch eine geeignete Variation der Mischungsverhältnisse der Ausgangssubstanz oder durch die Temperaturführung der Reaktion. So entstehen beispielsweise bei höheren Temperaturen aus den Carbonyl- bzw Thiocarbonylimidazoliden vermehrt die entsprechenden Carbonyle bzw. Thiocarbonyle.

Bei der Verfahrensvariante d) verfährt man am besten so, daß man Elaiophylin oder ein Elaiophylin-Derivat in äquimolaren Mengen oder in bis zu einem 5-fachen Überschuß mit Carbonyldiimidazol oder Thiocarbonyldiimidazol bis zur Beendigung der Reaktion, gegebenenfalls in Gegenwart einer Base wie Pyridin, umsetzt.

Eine Variante des Verfahrens besteht darin, daß man in einem geeigneten Lösungsmittel wie Chloroform, Methylenchlorid, THF, Essigester oder Dioxan arbeit. Auch hier kann ein Überschuß an Carbonyldiimidazol

oder Thiocarbonyldiimidazol, der bis zur etwa 5-fachen Menge betragen kann, angewandt werden. Die Reaktionstemperaturen liegen dabei zwischen 0 °C und +100 °C, vorzugsweise bei Verwendung eines Lösungsmittels zwischen dem Festpunkt und dem Siedepunkt des Lösungsmittels, insbesondere zwischen 0 °C und 40 °C. Die Reaktionszeiten betragen 1 bis 48 Stunden, bevorzugt 1 bis 24 Stunden. Die Beendigung der Reaktion kann beispielsweise mittels DC-Kontrolle bestimmt werden.

Die Ausgangsverbindungen für die Verfahrensvariante d), Carbonyldiimidazol bzw. Thiocarbonyldiimidazol, sind käuflich.

Mit Hilfe der Verfahrensvarienten e) lassen sich, wie oben bereits beschrieben, die OH-Gruppen in 9- und/oder 9′-Stellung verestern. Voraussetzung für eine Veresterung in dieser Position ist, daß bereits die Protonen der OH-Gruppen in 3″-/4‴-Position und 3″-4‴-Position substituiert sind. Anderenfalls würden zuerst diese OH-Gruppen mit dem Acylierungsagenz reagieren und erst später - bei entsprechendem Überschuß - die in 9-/9′-Position. Die Substituenten $R^2$ und $R^3$ können beispielsweise auf eine der beschriebenen Verfahrensvarianten a) bis d) in das Molekül eingeführt worden sein.

Sämtliche Verfahrensparameter für Variante e) sind der Beschreibung für Variante a) zu entnehmen. In manchen Fällen - besonders wenn $R^2/R^3$ und $R^4$ unterschiedliche Substituenten darstellen sollen - ist es zweckmäßig, im Anschluß an eine der Verfahrensvarianten a) bis d) das Reaktionsprodukt zu isolieren und gegebenenfalls zu reinigen, bevor man es gemäß Verfahrensvariante e) weiter umsetzt.

Die benötigten Ausgangsverbindungen der Formel IX und IX′ sind entweder käuflich oder können nach der für Variante a) beschriebenen Weise einfach synthetisiert werden.

Mit der Verfahrensvarianten f) läßt sich der Substituent $R^5$ in das Molekül einführen. Dabei läßt sich diese Reaktion unabhängig von der Art der Substituenten $R^2$, $R^3$ und $R^4$, selektiv an der OH-Gruppe der C11-C11′-Position durchführen.

Bei dieser Verfahrensvarianten geht man am besten so vor, daß man Elaiophylin oder ein entsprechendes $R^2$,$R^3$,$R^4$-Derivat mit einem Überschuß an einem Alkohol der Formel $HO-(CH_2)_n-R^5$ in Gegenwart katalytischer Mengen einer Lewissäure bis zur Beendigung der Reaktion umsetzt. Eine Variante des Verfahrens besteht darin, daß man in einem geeigneten Lösungsmittel wie Chloroform, Methylenchlorid, THF, Essigester oder Dioxan arbeitet. Als Lewissäure eignen sich beispielsweise Kupfer-, Eisen-oder Lithium-Halogenide, insbesondere $CuCl_2$, $FeCl_3$ oder LiBr.

Die Konzentration der Lewissäure - bezogen auf Elaiophylin oder das Elaiophylinderivat - beträgt 0,1 bis 5 Gew.-%, bevorzugt 0,5 bis 1 Gew.-%. Die Reaktionstemperaturen liegen dabei zwischen -40 °C und +100 °C, insbesondere zwischen 0 °C und 30 °C, vorzugsweise bei Verwendung eines Lösungsmittels zwischen dem Festpunkt und dem Siedepunkt des Lösungsmittels, insbesondere zwischen 0 °C und 30 °C. Die Reaktionszeiten betragen 1 bis 180 Minuten, bevorzugt 5 bis 60 Minuten. Die Beendigung der Reaktion kann beispielsweise mittels Dünnschichtchromatographie bestimmt werden.

Mit Hilfe der Verfahrensvarianten g) läßt sich das Halbacetal des Elaiophylins reduzieren, wobei der 6-Ring an C11 bzw. C11′ geöffnet wird. Diese Reduktion läßt sich jedoch nur dann durchführen, wenn $R^5$ Wasserstoff bedeutet. Mit Ausnahme des Falles, daß $R^2$ und $R^3$ ein Carbonylimidazolid, ein Thiocarbonylimidazolid oder zusammen einen Carbonyl- oder Thiocarbonylrest darstellen, ist die Art der übrigen Substituenten $R^2$, $R^3$ und $R^4$ für die Reduktion unkritisch.

Bei der Verfahrensvarinaten g) geht man am besten so vor, daß man Elaiophylin oder ein nach den Verfahrensvarianten a) bis f) oder i) erhältliches Elaiophylinderivat mit einem Alkali- oder Erdalkaliboranat, bevorzugt mit $NaBH_4$ in einem Lösungsmittel, bevorzugt in einem Alkohol wie Isopropanol oder in einem Ether wie THF, reduziert. Entsprechende Methoden sind beispielsweise beschrieben in Houben-Weyl, 4. Auflage, Band 4/1d, G. Thieme Verlag, Stuttgart 1981.

Die Reaktionstemperaturen liegen dabei zwischen -70 °C und +100 °C, vorzugsweise zwischen dem Festpunkt und dem Siedepunkt des Lösungsmittels, insbesondere zwischen -20 °C und +20 °C. Die Reaktionszeiten betragen 1 bis 60 Stunden, bevorzugt 5 bis 20 Stunden. Die Beendigung der Reaktion kann beispielsweise mittels DC-Kontrolle bestimmt werden.

Mittels der Verfahrensvarianten h) lassen sich die Hydroxygruppen an C11/C11′ und C15/15′ verestern. Hierbei kommt wiederum die Verfahrensvariante a) zur Awendung, was aber gleichzeitig bedeutet, daß vor der Veresterung der C15/C15′- und C11/C11′-OH-Gruppen zunächst die OH-Gruppen in 3″3‴- und 2″2‴-Position und gegebenenfalls auch die in 9/9′-Position verestert werden müßten, da andernfalls diese OH-Gruppen in einem Zuge mit den C15/15′- und C11 11′-OH-Gruppen verestert würden.

Sämtliche Verfahrensparameter für Variante h) sind der Beschreibung für Variante a) zu entnehmen. In manchen Fällen, besonders wenn $R^2$, $R^3$, $R^4$ und $R^7$ unterschiedliche Substituenten darstellen sollen, ist es zweckmäßig, im Anschluß an eine oder mehrere der Verfahrensvarianten a) bis e) und g) die Reaktionsprodukte zu isolieren und gegebenenfalls zu reinigen, bevor man das gewünschte Produkt gemäß Verfahrensvariante h) weiter umsetzt.

Mit Hilfe der Verfahrensvariante i) lassen sich die C-C-Doppelbindungen des Macrodiolidrings hydrieren. Diese Hydrierung kann sowohl vor als auch nach den Umsetzungen gemäß einer oder mehrerer der Verfahrensvariaten a) bis h) erfolgen. Sofern das gewünschte Elaiophilinderivat ungesättigte oder reduzierbare Substituenten $R^2$, $R^3$, $R^4$, $R^5$ oder $R^7$ enthalten soll, erfolgt die Hydrierung des Macordiolidrings zweckmäßigerweise vor der Ankoppelung des/der entsprechenden ungesättigten Substituenten.

Bei der Verfahrensvarianten i) geht man am besten so vor, daß man das zu hydrierende Elaiophylin oder Elaiophilinderivat, bevorzugt gelöst in einem Lösungsmittel wie Methanol, Ethanol, Isopropanol oder Ethylacetat oder einem Gemisch dieser Lösungsmittel oder einem wäßrigen Gemisch dieser Lösungsmittel, in Gegenwart eines gängigen Hydrierkatalysators nach literaturbekannten Verfahren mit Wasserstoff umsetzt. Gängige Hydrierkatalysatoren sind beispielsweise Elemente der 8. Gruppe wie Platin, Palladium oder auch Nickel, die meist zum Zwecke der Vergrößerung der reaktiven Oberfläche, beispielsweise auf Aktivkohle-, Siliziumdioxid- oder Aluminiumoxidträgern aufgetragen sind. Wird die Reaktion in einem absoluten primären Alkohol als Lösungsmittel durchgeführt, so erhält man außer einer Hydrierung der C=C-Doppelbindungen auch eine Ketalisierung zu den $C_{11}$; $C_{11'}$-Di-O-Alkylen.

Je nach verwendetem Katalysator kann die Reaktion sowohl ohne als auch mit Überdruck an Wasserstoff, beispielsweise bis zu 1 Atmosphäre, durchgeführt werden. Die Reaktionstemperaturen liegen zwischen 0°C und 40°C, vorzugsweise bei Raumtemperatur. Die Reaktionszeiten sind abhängig von der Ansatzgröße und der Konzentration der zu reduzierenden Verbindung. Solche Hydrierungsverfahren sind beispielsweise beschrieben in Organikum, Organisch Chemisches Grundpraktikum, 15. Auflage, VEB Deutscher Verlag der Wissenschaften, Berlin, 1976, S. 359-371.

Die Reinigung, Isolierung und Aufarbeitung der Substanzen erfolgt nach den üblichen Methoden: beispielsweise können die Reaktionsprodukte durch Chromatographie an polaren Trägermaterialien wie Kieselgel oder ®Sephadex LH 20 mit Lösungsmitteln wie niederen Alkanolen wie Methanol oder Chloroform oder Essigester oder Methanol/Chloroform-Mischungen aber auch durch extraktive Methoden wie flüssig/flüssig-Extraktion oder fest/flüssig-Extraktion oder durch Kristallisation gereinigt werden.

Die Derivate des Elaiophylins zeigen anthelmintische Wirkung, insbesondere gegen Haemonchus, Trichostrongylus, Ostertagia, Cooperia, Chabertia, Strongyloides, Oesophagostomum, Hyostrongylus, Ancylostoma, Ascaris und Heterakis. Besonders ausgeprägt ist die Wirksamkeit gegenüber Magen-Darm-Strongyliden und Lungenwürmern, von denen vor allem Haus- und Nutztiere befallen werden.

Die Elaiophylinderivate können grundsätzlich als solche in Substanz verabreicht werden. Bevorzugt ist die Verwendung in Mischung mit geeignetem Trägermaterial. Als Trägermaterial können die üblichen Futtermittelmischungen verwendet werden.

In den sich anschließenden Beispielen wird die Erfindung weiter erläutert. Prozentangaben beziehen sich auf das Gewicht und Mischungsverhältnisse bei Flüssigkeiten beziehen sich auf das Volumen, wenn keine anderen Angaben gemacht wurden.

**Beispiele**

Allgemeine Verfahrensvorschriften

**Verfahren 1**

1 mmol Macrodiolid vom Elaiophylin-Typ (hierunter sind Verbindungen der Formel I zu verstehen, in denen der Macrodiolidring hydriert oder nicht hydriert ist) werden in 10 ml Chloroform und 10 ml Pyridin gelöst und mit dem entsprechenden Carbonsäurederivat gerührt. Der Überschuß an Carbonsäurederivat wird mit 50 ml Wasser zerstört. Das Produkt wird durch dreimalige Extraktion mit Ethylacetat in die organische Phase überführt. Die organische Phase wird mit 0,1 N Salzsäure und Wasser gewaschen, über Natriumsulfat getrocknet und im Vakuum eingeengt. Durch Chromatographie an 100 g Kieselgel mit einem linearen Gradienten Ethylacetat : Hexan : 1 : 5 auf Ethylacetat (ca. 5 l) werden die entsprechenden Verbindungen erhalten (s. Tabelle 1 und 2).

**Verfahren 2**

1 mmol Macrodiolid vom Elaiophylin-Typ werden in 10 ml Alkohol gelöst und mit 70 mg $FeCl_3$ bei

Raumtemperatur gerührt. Nach der Zugabe von 100 ml Ethylacetat wird zweimal mit 50 ml einer gesättigten EDTA-Lösung (pH = 7, mit NaOH einstellen) und zweimal mit 50 ml Wasser gewaschen. Nach dem Trocknen der organischen Phase über Natriumsulfat wird im Vakuum bis zu einem festen Rückstand eingeengt.

**Verfahren 3**

1 mmol Macrodiolid vom Elaiophylin-Typ wird in 100 ml Isopropanol mit 250 mg $NaBH_4$ für 24 Stunden bei Raumtemperatur gerührt. Der Überschuß an Reduktionsmittel wird durch Zugabe von Aceton zerstört. Das Lösungsmittel wird im Vakuum abdestilliert und der Rückstand in Ethylacetat aufgenommen. Nach dem Ausschütteln mit Wasser wird die organische Phase über Natriumsulfat getrocknet und im Vakuum abdestilliert. Chromatographie an 100 g Kieselgel, mit einem linearen Gradienten von Ethylacetat : Hexan 1 : 5 auf Ethylacetat (bei Verbindungen mit $R_1 = R_1'$ und $R_2$, $R_3 \neq H$) bzw. Chloroform : Methanol 40 : 1 auf Chloroform : Methanol 1 : 1 (bei Verbindungen mit $R_1 = R_1'$ und $R_2$, $R_3$ = H) ergibt die reduzierten Verbindungen.

**Verfahren 4**

1 mmol der Verbindung aus Beispiel 22 (s. Tabelle 1) werden in 20 ml Methylenchlorid suspendiert und unter Stickstoff mit 6 mmol SEM-Chlorid oder MEM-Chlorid und 2 ml (12 mmol) Diisopropylethylamin für 20 Stunden bei 25 °C gerührt. Nach der Zugabe von 5 ml Methanol wird das Lösungsmittel im Vakuum abdestilliert. Der Rückstand wird in Methylenchlorid aufgenommen und mit Wasser ausgeschüttelt. Nach dem Trocknen über Natriumsulfat wird das Lösungsmittel im Vakuum abgezogen. Chromatographie an Kieselgel (100 g) mit einem Linearen Gradienten von Ethylacetat : Hexan / 1 : 5 auf Ethylacetat (ca. 5 l) ergibt die entsprechenden Verbindungen.

**Verfahren 5**

1 mmol Macrodiolid vom Elaiophylin-Typ wird in 10 ml Dichlormethan und 10 ml Pyridin gelöst. Nach Zugabe eines Überschusses an Carbonsäureanhydrid und 1 mmol 4-Dimethylaminopyridin rührt man die Mischung bei Raumtemperatur bis die Reaktion laut Dünnschichtchromatogramm beendet ist (ca. 2 bis 6 Stunden). Überschüssiges Anhydrid wird durch Zugabe von 5 ml Methanol unter Eiskühlung zerstört. Man verdünnt mit 50 ml Ethylacetat, extrahiert erst dreimal mit je 20 ml gesättigter Natriumhydrogencarbonatlösung, dann mit gerade soviel 0,1 N HCl-Lösung, daß die wäßrige Phase den pH-Wert 5 nicht überschreitet, und schließlich noch einmal mit 20 ml der gesättigten Natriumhydrogencarbonatlösung. Nach Trocknung der organischen Phase über Natriumsulfat und Einengen im Vakuum wird das Produkt entweder durch Kristallisation oder durch Säulenchromatographie an Kieselgel isoliert.

**Verfahren 6**

1 mmol Macrodiolid vom Elaiophylin-Typ wird mit einem Überschuß des betreffenden Aldehyds oder Ketons, die ihrerseits auch als Lösungsmittel verwendet werden können, bei Raumtemperatur, gegebenenfalls unter Verdünnung mit einem Cosolvens wie Dichlormethan, Chloroform oder Tetrahydrofuran, in Gegenwart katalytischer Mengen einer wasserfreien Lewis-Säure wie $ZnCl_2$, $ZnI_2$, $FeCl_3$, $CuCl_2$, $CuSO_4$ gerührt, bis die Reaktion laut Dünnschichtchromatogramm beendet ist. Nach Verdünnen mit 50 ml Ethylacetat und dreimaligem Waschen mit je 20 ml einer gesättigten Natriumhydrogencarbonatlösung isoliert man das Produkt durch Säulenchromatographie an Kieselgel.

**Verfahren 7**

1 mmol Macrodiolid vom Elaiophylin-Typ wird in einem geeigneten Lösungsmittel oder Lösungsmittelgemisch gelöst und mit 10 Gew.-% Hydrierkatalysator, bevorzugt 10 % Palladium an Tierkohle, versetzt. Ein bewährtes Lösungsmittelsystem besteht in einer Mischung von Isopropanol/Ethylacetat/Wasser im

Volumenverhältnis 4:3:2. Die Hydrierung erfolgt bei Raumtemperatur unter Magnetrührung oder Schütteln in einer geschlossenen Hydrierapparatur unter Einhaltung eines leichten Wasserstoffüberdrucks von bis zu 0,2 bar. Unter diesen Bedingungen ist die Reaktion innerhalb 0,5 bis 2 Stunden unter Aufnahme von 4 mmol $H_2$ beendet. Nach Abfiltrieren des Katalysators und Einengen muß das Produkt in einigen Fällen noch durch Umkristallisieren oder Säulenchromatographie gereingt werden.

**Verfahren 8**

1 mmol Macrodiolid vom Elaiophylin-Typ wird in 10 bis 100 ml wasserfreiem Methanol gelöst und mit bis zu 40 Gew.-% getrocknetem Hydrierkatalysator, bevorzugt 10 % Palladium an Tierkohle versetzt. Die Hydrierung und Produktisolierung erfolgt genau wie bei Verfahren 7 beschrieben.

In den nachfolgenden Tabellen sind die gemäß den vorstehend beschriebenen Verfahren 1 bis 8 synthetisierten Verbindungen aufgeführt. Die Substituenten im Rest $R^{1'}$ wurden mit $R^{2'}$, $R^{3'}$, $R^{4'}$, $R^{5'}$ und $R^{7'}$ bezeichnet. In Tabelle 1 sind die verschiedenen Substituenten der Elaiophylin-Derivate zwecks Identifizierung der Verbindungen angegeben. In Tabelle 2 ist das jeweilige Herstellungsverfahren, die individuellen Verfahrensparameter, die Art des Reagenzes, die relative Konzentration des Reagenzes (bezogen auf die Ausgangsverbindung vom Elaiophylin-Typ), die Ausbeute sowie ausgewählte, charakteristische analytische Daten der erhaltenen Verbindungen angegeben. Eine Erklärung der in den Tabellen verwendeten Ausdrücke findet sich am Ende.

**Tabelle 1**

| Verbindung | Vorstufe | Hydr. | $R^1$ | $R^2$ | $R^3$ | $R^4$ | $R^5$ | $R^7$ | $R^{1'}$ | $R^{2'}$ | $R^{3'}$ | $R^{4'}$ | $R^{5'}$ | $R^{7'}$ |
|---|---|---|---|---|---|---|---|---|---|---|---|---|---|---|
| 1 | Ela | - | II | Ac | Ac | H | H | - | II | Ac | Ac | H | H | - |
| 2 | Ela | - | II | Ac | Ac | H | H | - | II | H | Ac | H | H | - |
| 3 | Ela | - | II | H | Ac | H | H | - | II | H | Ac | H | H | - |
| 4 | Ela | - | II | H | Ac | H | H | - | II | H | H | H | H | - |
| 5 | Ela | - | II | p-Br-Bz | p-Br-Bz | H | H | - | II | p-Br-Bz | p-Br-Bz | H | H | - |
| 6 | Ela | - | II | p-Br-Bz | p-Br-Bz | H | H | - | II | H | p-Br-Bz | H | H | - |
| 7 | Ela | - | II | H | p-Br-Bz | H | H | - | II | H | p-Br-Bz | H | H | - |
| 8 | Ela | - | II | H | p-Br-Bz | H | H | - | II | H | H | H | H | - |
| 9 | Ela | - | II | Val | Val | H | H | - | II | Val | Val | H | H | - |
| 10 | 1 | - | II | Ac | Ac | H | $CH_3$ | - | II | Ac | Ac | H | $CH_3$ | - |
| 11 | 1 | - | II | Ac | Ac | H | $C_2H_5$ | - | II | Ac | Ac | H | $C_2H_5$ | - |
| 12 | 2 | - | II | Ac | Ac | H | $CH_3$ | - | II | H | Ac | H | $CH_3$ | - |
| 13 | Ela | - | II | 2-$C_5H_3O_2$ | 2-$C_5H_3O_2$ | H | H | - | II | 2-$C_5H_3O_2$ | 2-$C_5H_3O_2$ | H | H | - |
| 14 | 13 | - | II | 2-$C_5H_3O_2$ | 2-$C_5H_3O_2$ | H | $CH_3$ | - | II | 2-$C_5H_3O_2$ | 2-$C_5H_3O_2$ | H | $CH_3$ | - |
| 15 | Ela | - | II | >C=O | | H | H | - | II | >C=O | | H | H | - |
| 16 | Ela | - | II | >C=O | | H | H | - | II | C(O)Im | C(O)Im | H | H | - |
| 17 | 1 | - | II | Ac | Ac | H | H | - | III | Ac | Acn | H | - | H |
| 18 | 1 | - | III | Ac | Ac | H | - | H | III | Ac | Ac | H | - | H |
| 19 | 9 | - | II | Val | Val | H | H | - | III | Val | Val | H | - | H |
| 20 | 9 | - | III | Val | Val | H | - | H | III | Val | Val | H | - | H |
| 21 | 17 | - | II | Ac | Ac | H | $CH_3$ | - | III | Ac | Ac | H | - | H |
| 22 | Ela | - | II | H | H | H | $CH_3$ | - | II | H | H | H | $CH_3$ | - |
| 23 | 22 | - | II | SEM | SEM | H | $CH_3$ | - | II | SEM | SEM | H | $CH_3$ | - |

**Fortsetzung Tabelle 1**

| Verbindung | Vorstufe | Hydr. | $R^1$ | $R^2$ | $R^3$ | $R^4$ | $R^5$ | $R^7$ | $R^{1'}$ | $R^2$ | $R^3$ | $R^4$ | $R^5$ | $R^7$ |
|---|---|---|---|---|---|---|---|---|---|---|---|---|---|---|
| 24 | 22 | - | II | MEM | MEM | H | $CH_3$ | - | II | MEM | MEM | H | $CH_3$ | - |
| 25[t)] | Ela | - | II | Ac | Ac | Ac | Ac | - | II | Ac | Ac | Ac | Ac | - |
| 26 | Ela | - | II | Mes | Mes | H | H | - | II | Mes | Mes | H | H | - |
| 27 | Ela | - | II | >$C(CH_3)_2$ | | H | H | - | II | >$C(CH_3)_2$ | | H | H | - |
| 28 | 27 | - | II | >$C(CH_3)_2$ | | H | $CH_3$ | - | II | >$C(CH_3)_2$ | | H | $CH_3$ | - |
| 29 | Ela | - | II | Bz | Bz | H | H | - | II | Bz | Bz | H | H | - |
| 30 | 29 | - | II | Bz | Bz | H | $CH_3$ | - | II | Bz | Bz | H | $CH_3$ | - |
| 31 | Ela | + | II | H | H | H | H | - | II | H | H | H | H | - |
| 32 | 1 | + | II | Ac | Ac | H | H | - | II | Ac | Ac | H | H | - |
| 33 | 2 | + | II | Ac | Ac | H | H | - | II | H | Ac | H | H | - |
| 34 | 3 | + | II | H | Ac | H | H | - | II | H | Ac | H | H | - |
| 35 | 4 | + | II | H | Ac | H | H | - | II | H | H | H | H | - |
| 36 | 5 | + | II | p-Br-Bz | p-Br-Bz | H | H | - | II | p-Br-Bz | p-Br-Bz | H | H | - |
| 37 | 6 | + | II | p-Br-Bz | p-Br-Bz | H | H | - | II | H | p-Br-Bz | H | H | - |
| 38 | 7 | + | II | H | p-Br-Bz | H | H | - | II | H | p-Br-Bz | H | H | - |
| 39 | 8 | + | II | H | p-Br-Bz | H | H | - | II | H | H | H | H | - |
| 40 | 31 | + | II | Val | Val | H | H | - | II | Val | Val | H | H | - |
| 41 | 53 | + | II | Ac | Ac | H | $CH_3$ | - | II | Ac | Ac | H | $CH_3$ | - |
| 42 | 32 | + | II | Ac | Ac | H | $C_2H_5$ | - | II | Ac | Ac | H | $C_2H_5$ | - |
| 43 | 33 | + | II | Ac | Ac | H | $CH_3$ | - | II | H | Ac | H | $CH_3$ | - |
| 44 | 31 | + | II | 2-$C_5H_3OS$ | 2-$C_5H_3OS$ | H | H | - | II | 2-$C_5H_3OS$ | 2-$C_5H_3OS$ | H | H | - |
| 45 | 44 | + | II | 2-$C_5H_3OS$ | 2-$C_5H_3OS$ | H | $CH_3$ | - | II | 2-$C_5H_3OS$ | 2-$C_5H_3OS$ | H | $CH_3$ | - |
| 46 | 31 | + | II | >C=O | | H | H | - | II | >C=O | | H | H | - |
| 47 | 31 | + | II | >C=O | | H | H | - | II | C(O)Im | C(O)Im | H | H | - |

Fortsetzung Tabelle 1

| Verbindung | Vorstufe | Hydr. | $R^1$ | $R^2$ | $R^3$ | $R^4$ | $R^5$ | $R^7$ | $R^{1'}$ | $R^2$ | $R^3$ | $R^4$ | $R^5$ | $R^7$ |
|---|---|---|---|---|---|---|---|---|---|---|---|---|---|---|
| 48 | 32 | + | II | Ac | Ac | H | H | - | III | Ac | Ac | H | - | H |
| 49 | 32 | + | III | Ac | Ac | H | - | H | III | Ac | Ac | H | - | H |
| 50 | 40 | + | II | Val | Val | H | H | - | III | Val | Val | H | - | H |
| 51 | 40 | + | III | Val | Val | H | - | H | III | Val | Val | H | - | H |
| 52 | 48 | + | II | Ac | Ac | H | $CH_3$ | - | III | Ac | Ac | H | - | H |
| 53 | Ela | + | II | H | H | H | $CH_3$ | - | II | H | H | H | $CH_3$ | - |
| 54 | 31 | + | II | SEM | SEM | H | H | - | II | SEM | SEM | H | H | - |
| 55 | 31 | + | II | MEM | MEM | H | H | - | II | MEM | MEM | H | H | - |
| 56 | 25 | + | II | Ac | Ac | Ac | Ac | - | II | Ac | Ac | Ac | Ac | - |
| 57 | 31 | + | II | Bz | Bz | H | H | - | II | Bz | Bz | H | H | - |
| 58 | 57 | + | II | Bz | Bz | H | $CH_3$ | - | II | Bz | Bz | H | $CH_3$ | - |

Tabelle 2

| Verbindung | Verfahren | Reaktions-zeit (h) | Reaktions-temp. (°) | Reagenz | Reagenz-Äquivalente | Ausbeute (%) | Ausgewählte analytische Daten tt): Fp. (°C) | $[\alpha_D^{20}]$ (C=1, $CH_3OH$) | FAB-MS $MNa^+$ | Elementaranalyse C (ber.) | C (gef.) | H (ber.) | H (gef.) |
|---|---|---|---|---|---|---|---|---|---|---|---|---|---|
| 1 | 1 | 24 | 20 | $Ac_2O$ | 10 | 97 | 186 | | 1215 | | | | |
| 2 | 1 | 12 | -20 | $Ac_2O$ | 5 | 62 | | | 1173 | | | | |
| 3 | 1 | 14 | -20 | $Ac_2O$ | 2,5 | 66 | | | 1131 | | | | |
| 4 | 1 | 8 | -20 | $Ac_2O$ | 1,5 | 46 | | | 1089 | | | | |
| 5 | 1 | 10 | 20 | p-BrBzCl | 5 | 64 | 194 | | | | | | |
| 6 | 1 | 3 | 20 | p-BrBzCl | 3,3 | 33 | 182 | | | | | | |
| 7 | 1 | 3 | - 5 | p-BrBzCl | 2,6 | 54 | 205 | | | | | | |
| 8 | 1 | 5 | -35 | p-BrBzCl | 1,5 | 32 | 160 | | | | | | |
| 9 | 1 | 90 | 20 | $Val_2O$ | 8 | 74 | 173 | | | | | | |
| 10 | 2 | 0,5 | 20 | $CH_3OH$ | Solv. | 82 | | | 1243 | | | | |
| 11 | 2 | 1 | 20 | $C_2H_5OH$ | Solv. | 79 | | | 1271 | | | | |
| 12 | 2 | 0,5 | 20 | $CH_3OH$ | Solv. | 81 | | | 1201 | | | | |
| 13 | 5 | 6 | 20 | $(2\text{-}C_5H_3O_2)_2O$ | 12 | 83 | | | 1423 | | | | |
| 14 | 2 | 0,5 | 20 | $CH_3OH$ | Solv. | 90 | | | 1451 | | | | |
| 15 | 1 | 72 | 20 | $Im_2CO$ | 5 | 37 | 207 | + 3° | | | | | |
| 16 | 1 | 72 | 20 | $Im_2CO$ | 5 | 35 | 160 | -42° | | | | | |
| 17 | 3 | 12 | 20 | $NaBH_4$ | 6,5 | 33 | | | 1217 | | | | |
| 18 | 3 | 12 | 20 | $NaBH_4$ | 6,5 | 42 | | | 1219 | | | | |
| 19 | 3 | 12 | 20 | $NaBH_4$ | 6,5 | 30 | | | 1385 | | | | |
| 20 | 3 | 12 | 20 | $NaBH_4$ | 6,5 | 50 | | | 1387 | | | | |
| 21 | 2 | 0,5 | 20 | $CH_3OH$ | Solv. | 80 | | | 1231 | | | | |
| 22 | 2 | 0,5 | 20 | $CH_3OH$ | Solv. | 95 | | | 1075 | | | | |

Fortsetzung Tabelle 2

| Verbindung | Verfahren | Reaktions-zeit (h) | Reaktions-temp. (°) | Reagenz | Reagenz-Äquivalente | Ausbeute (%) | Fp. (°C) | $[\alpha_D^{20}]$ (C=1, $CH_3OH$) | FAB-MS $MNa^+$ | C (ber.) | C (gef.) | H (ber.) | H (gef.) |
|---|---|---|---|---|---|---|---|---|---|---|---|---|---|
| | | | | | | | Ausgewählte analytische Daten tt) | | | Elementaranalyse | | | |
| 23 | 4 | 24 | 20 | SEMCl | 8 | 35 | | | 1595 | | | | |
| 24 | 4 | 24 | 20 | MEMCl | 8 | 30 | | | 1427 | | | | |
| 25 t) | 5 | 12 | 20 | $Ac_2O$ | 40 | 70 | | +10° | | 61,75 | 61,55 | 7,69 | 7,65 |
| 26 | 1 | 1 | 20 | MesCl | 5 | 41 | | | 1359 | | | | |
| 27 | 6 | 14 | 30 | $AcCH_3$ | 100 | 20 | | | 1127 | | | | |
| 28 | 2 | 0,5 | 20 | $CH_3OH$ | Solv. | 90 | | | 1155 | | | | |
| 29 | 5 | 6 | 20 | $Bz_2O$ | 12 | 86 | | -96° | | | | | |
| 30 | 2 | 0,5 | 20 | $CH_3OH$ | Solv. | 79 | | | | 68,64 | 68,72 | 7,40 | 7,60 |
| 31 | 7 | 2 | 20 | $H_2/Pd$ | 4 | 89 | 198 | -77° | | | | | |
| 32 | 7 | 1,5 | 20 | $H_2/Pd$ | 4 | 90 | 125 | -86° | | | | | |
| 33 | 7 | 1,5 | 20 | $H_2/Pd$ | 4 | 75 | | | 1181 | | | | |
| 34 | 7 | 2 | 20 | $H_2/Pd$ | 4 | 70 | | | 1139 | | | | |
| 35 | 7 | 2 | 20 | $H_2/Pd$ | 4 | 86 | | | 1097 | | | | |
| 36 | 7 | 3 | 20 | $H_2/Pd$ | 4 | 71 | | | | 55,79 | 56,95 | 6,16 | 6,27 |
| 37 | 7 | 2,5 | 20 | $H_2/Pd$ | 4 | 79 | | | | 56,93 | 56,72 | 6,69 | 6,89 |
| 38 | 7 | 3 | 20 | $H_2/Pd$ | 4 | 86 | | | | 58,37 | 58,12 | 7,35 | 7,50 |
| 39 | 7 | 3 | 20 | $H_2/Pd$ | 4 | 80 | | | | 60,23 | 60,04 | 8,20 | 8,44 |
| 40 | 1 | 72 | 20 | $Val_2O$ | 8 | 77 | | | 1391 | | | | |
| 41 | 1 | 18 | 20 | $Ac_2O$ | 20 | 82 | | -53° | | | | | |
| 42 | 2 | 1 | 20 | $C_2H_5OH$ | Solv. | 89 | | | 1279 | | | | |
| 43 | 2 | 0,5 | 20 | $CH_3OH$ | Solv. | 84 | | | 1209 | | | | |
| 44 | 5 | 8 | 20 | $(2\text{-}C_5H_3OS)_2O$ | 12 | 79 | | | | 60,30 | 59,99 | 7,11 | 6,89 |
| 45 | 2 | 0,5 | 20 | $CH_3OH$ | Solv. | 90 | | | | 60,77 | 60,57 | 7,25 | 7,39 |

Fortsetzung Tabelle 2

Ausgewählte analytische Daten tt)

| Verbindung | Verfahren | Reaktions-zeit (h) | Reaktions-temp. (°) | Reagenz | Reagenz-Äquivalenz | Ausbeute (%) | Fp. (°C) | $[\alpha_D^{20}]$ (C=1, $CH_3OH$) | FAB-MS $MNa^+$ | Elementaranalyse C (ber.) | C (gef.) | H (ber.) | H (gef.) |
|---|---|---|---|---|---|---|---|---|---|---|---|---|---|
| 46 | 1 | 72 | 20 | $Im_2CO$ | 5 | 39 | | | | 61,97 | 62,20 | 8,54 | 8,33 |
| 47 | 1 | 72 | 20 | $Im_2CO$ | 5 | 31 | | | | 60,65 | 61,00 | 7,92 | 7,69 |
| 48 | 3 | 12 | 20 | $NaBH_4$ | 6,5 | 28 | | | 1225 | | | | |
| 49 | 3 | 12 | 20 | $NaBH_4$ | 6,5 | 44 | | | 1227 | | | | |
| 50 | 3 | 12 | 20 | $NaBH_4$ | 6,5 | 39 | | | 1393 | | | | |
| 51 | 3 | 12 | 20 | $NaBH_4$ | 6,5 | 54 | | | 1395 | | | | |
| 52 | 2 | 0,5 | 20 | $CH_3OH$ | Solv. | 85 | | | 1239 | | | | |
| 53 | 8 | 22 | 20 | $H_2/Pd$ | 4 | 87 | 114 | -49,5 | | | | | |
| 54 | 4 | 24 | 20 | SEMCl | 8 | 32 | | | 1603 | | | | |
| 55 | 4 | 24 | 20 | MEMCl | 8 | 38 | | | 1435 | | | | |
| 56t) | 8 | 2 | 20 | $H_2/Pd$ | 4 | 77 | | | | 61,38 | 61,30 | 8,24 | 8,30 |
| 57 | 5 | 6 | 20 | $Bz_2O$ | 20 | 73 | 190 | -106 | | | | | |
| 58 | 2 | 0,5 | 20 | $CH_3OH$ | Solv. | 83 | | | | 68,27 | 67,97 | 7,91 | 7,99 |

**Fußnoten zu Tabelle 1 und 2**

t) Die $^{13}C$-NMR-Spektren in Pyridin-$d^5$ belegen, daß die peracylierten Verbindungen als geöffnete Hemiacetale in den 11,11'-Diketo-15,15'-Di-O-Acylformen vorliegen: $\delta$ ($C^{11}$,$C^{11'}$) 210,1 ppm.

tt) Schmelzpunkte wurden nur von eindeutig kristallinen Substanzen bestimmt und sind nicht korrigiert.

Die Verbindungen schmelzen im allgemeinen unter Zersetzung. Alle übrigen Substanzen sind amorphe Feststoffe. Die Strukturzuordnung basiert insbesondere auf $^1H$-, $^{13}C$-, 2D-NMR-, FAB-MS- und IR-Spektroskopie.

**Legende zur Tabelle**

Ela: Elaiophylin $R^1$,$R^{1'}$ = II und $R^2$,$R^3$,$R^4$,$R^5$ = H
Ac: $-CO-CH_3$
p-Br-Bz: para-Brombenzoyl
Val: $-CO-(CH_2)_3CH_3$
Solv.: Reagenz = Lösungsmittel
$2-C_5H_3O_2$: 2-Furoyl
$Im_2CO$: Carbonyldiimidazol
Im: 1-Imidazolyl
SEM: $(CH_3)_3SiCH_2CH_2OCH_2-$
MEM: $CH_3OCH_2CH_2OCH_2-$
Mes: $-SO_2CH_3$
Bz: Benzoyl
$2-C_5H_3OS$: 2-Thienoyl
Hydr.: in dieser Spalte ist angegeben, ob der Macrodiolid-Ring hydriert ist (+) oder nicht (-)

II bzw. III: in den Spalten unter $R^1$ und $R^{1'}$ ist angegeben, welchem Formeltyp (s. Formel I) die Substituenten $R^1$ und $R^{1'}$ angehören.

**Anthelminthische Wirkung der Elaiophylinderivate**

Die anthelminthische Wirkung der Elaiophylinderivate wurde an Lämmern mit 30 bis 40 kg Körpergewicht untersucht. Dazu wurden die Lämmer artifiziell mit Infektionsstadien von Labmagennematoden (Haemunchus contortus) infiziert. Nach Abschluß der Entwicklungszeit (Präpatenzperiode) der Nematoden erfolgte die Applikation der Elaiophylinderivate.

Durch koproskopische Untersuchungen vor und bis zu 14 Tagen nach der Applikation der Elaiophylinderivate und anschließender Sektion mit helminthologischer Aufarbeitung wurde die prozentuale Reduktion der Schaf-Nematoden ermittelt (s. Tabelle 3). Als Vergleichssubstanz diente Elaiophylin. Zusätzlich wurde auch noch die antibakterielle Aktivität der erfindungsgemäßen Elaiophylinderivate gegen Staph. aureus und Strept. pyogenes ermittelt. Überraschenderweise zeigen die erfindungsgemäßen Verbindungen keine oder nur sehr geringe antibakterielle Aktivität. Gerade deshalb eignen sich die erfindungsgemäßen Verbindungen insbesondere für den Einsatz als Anthelminthika, da hier eine antibakterielle Wirkung unerwünscht ist.

**Tabelle 3**

| Verabreichte Verbindung aus Beispiel | Dosierung (mg/kg) | Reduktion v. H. contortus (%) | antibakterielle Aktivität (µg/ml) gegen Stap. aureurs | Strept. pyogenes |
|---|---|---|---|---|
| Elaiophylin | 2,5 (s.c.) | 35-45 | 1,56 | 1,56 |
| | 5,0 (oral) | 70-95 | | |
| 1 | 2,5 (s.c.) | 70-90 | > 100 | > 100 |
| | 5,0 (oral) | 40-60 | | |
| 2 | 2,5 (s.c.) | 50-70 | > 100 | 6,25 |
| | 5,0 (oral) | 30-40 | | |
| 5 | 2,5 (s.c.) | 5-10 | > 100 | > 100 |
| | 5,0 (oral) | 5-10 | | |
| 8 | 2,5 (s.c.) | 50-70 | > 100 | 3,13 |
| | 5,0 (oral) | 10-20 | | |
| 9 | 2,5 (s.c.) | 50-70 | 50,0 | 50,0 |
| | 5,0 (oral) | 50-70 | | |
| 10 | 2,5 (s.c.) | 10-20 | > 100 | > 100 |
| | 5,0 (oral) | 30-50 | | |
| 15 | 2,5 (s.c.) | 5-10 | > 100 | > 100 |
| | 5,0 (oral) | 5-10 | | |
| 16 | 2,5 (s.c.) | 5-10 | > 100 | > 100 |
| | 5,0 (oral) | - | | |
| 17 | 2,5 (s.c.) | 30-50 | 25,0 | 25,0 |
| | 5,0 (oral) | 30-50 | | |
| 22 | 2,5 (s.c.) | 30-40 | 3,13 | 1,56 |
| | 5,0 (oral) | 60-75 | | |

Fortsetzung Tabelle 3

| Verabreichte Verbindung aus Beispiel | Dosierung (mg/kg) | Reduktion v. H. contortus (%) | antibakterielle Aktivität (µg/ml) gegen Stap. aureurs | Strept. pyogenes |
|---|---|---|---|---|
| 29 | 2,5 (s.c.) | 10-20 | > 100 | > 100 |
| | 5,0 (oral) | 60-80 | | |
| 31 | 2,5 (s.c.) | 30-40 | > 100 | > 100 |
| | 5,0 (oral) | 50-75 | | |
| 32 | 2,5 (s.c.) | 50-60 | > 100 | > 100 |
| | 5,0 (oral) | 40-60 | | |
| 41 | 2,5 (s.c.) | 40-60 | > 100 | > 100 |
| | 2,5 (oral) | 60-80 | | |
| 53 | 2,5 (s.c.) | 40-50 | 25 | 6,25 |
| | 3,8 (oral) | 60-75 | | |
| 57 | 2,5 (s.c.) | 20-30 | > 100 | > 100 |
| | 5,0 (oral) | 40-60 | | |

**Ansprüche**

1. Elaiophylinderivate der Formel I,

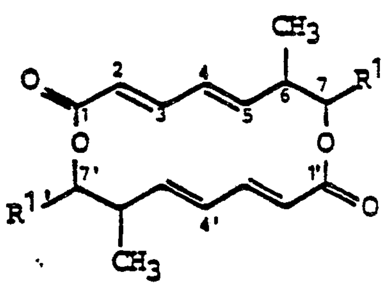

(I)

wobei die C-C-Doppelbindungen in dem Macrodiolidring der Verbindung der Formel I auch hydriert sein können und in der

R' ein Rest der Formel II oder III ist,

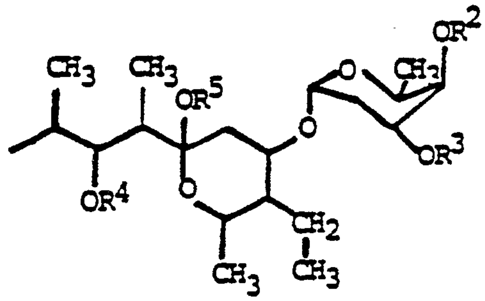

(II)

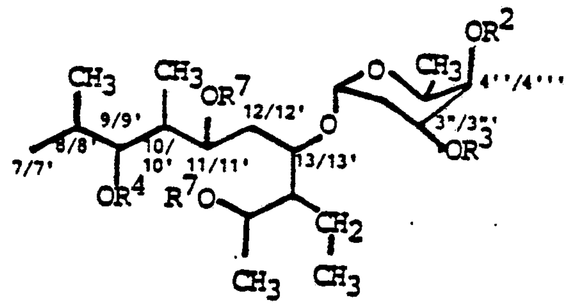

(III)

worin
$R^2$ und $R^3$ gleich oder verschieden sind und Wasserstoff oder einen Rest der Formel IV oder IV′

$-\overset{O}{\overset{\|}{C}}-R^6$ (IV)

$-\overset{O}{\overset{\|}{C}}-(CH_2)_n-R^6$ (IV′)

bedeuten, in der
n 1 bis 3 und
$R^6$ $C_1$-$C_{15}$-Alkyl, $C_2$-$C_{15}$-Alkenyl, $C_2$-$C_{15}$-Alkinyl, $C_3$-$C_9$-Cycloalkyl, Aryl- oder Heteroaryl bedeutet, wobei die Aryl- und Heteroarylreste gegebenenfalls halogen-, nitro-, cyano-, hydroxy-, $C_1$-$C_4$-alkyl- oder $C_1$-$C_4$-alkoxysubstituiert sind
und wobei für den Fall, daß $R^3$ Wasserstoff ist, $R^2$ auch Wasserstoff ist,
oder worin
$R^2$ und $R^3$ gleich sind und $C_1$-$C_4$-Alkyl, Benzyl, Allyl, MEM, MOM, SEM, Trimethylsilyl, Triethylsilyl, Dimethylphenylsilyl, Dimethylcyclohexylsilyl oder Dimethyltertiärbutylsilyl bedeutet
oder
$R^2$ und $R^3$ Sulfonsäureester der Formel $SO_2R^{10}$ bedeuten, in der
$R^{10}$ $C_1$-$C_{10}$-Alkyl, Phenyl oder p-Toloyl bedeutet
oder
$R^2$ und $R^3$ einen Rest der Formel V oder V′ darstellen,

$-\overset{X}{\overset{\|}{C}}-Z-R^{12}$ (V)

$-\overset{X}{\overset{\|}{C}}-Z-(CH_2)_n-R^{12}$ (V′)

in der

n 1 bis 3

X Sauerstoff oder Schwefel

$R^{12}$ $C_1$-$C_{15}$-Alkyl, $C_3$-$C_9$-Cycloalkyl, Aryl, Pyridyl, Pyrimidyl oder Pyrazinyl bedeutet, wobei die Aryl-, Pyridyl-, Pyrimidyl- oder Pyrazinylreste gegebenenfalls halogen-, nitro-, cyano- oder $C_1$-$C_4$-alkoxysubstituiert sind und

Z Sauerstoff, -N-H oder einen Rest -N-$R^{12}$ oder -N-$(CH_2)_n$-$R^{12}$ bedeutet, wobei n und $R^{12}$ die oben angegebenen Bedeutungen haben

oder worin

$R^2$ und $R^3$ zusammen einen Rest der Formel VI darstellen

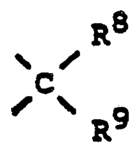 (VI)

in der

$R^8$ und $R^9$ gleich oder verschieden sind und Wasserstoff oder unverzweigtes $C_1$-$C_{10}$-Alykl bedeuten oder wobei

$R^8$ und $R^9$ eine Alkylenkette darstellt, die zusammen mit dem sie tragenden C-Atom einen 5-, 6- oder 7-gliedrigen Ring bildet

oder worin

$R^2$ und $R^3$ für den Fall, daß $R^1$ ein Rest der Formel II ist, gleich sind und einen Rest der Formel VIII darstellen,

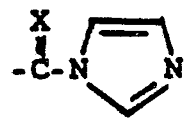 (VIII)

in der

X Sauerstoff oder Schwefel bedeutet

oder

$R^2$ und $R^3$ für den Fall, daß $R^1$ ein Rest der Formel II ist, zusammen einen Rest der Formel $>C=X$ darstellen, worin

X Sauerstoff oder Schwefel bedeutet

und worin

$R^4$ sofern $R^2$ und $R^3$ Wasserstoff sind, Wasserstoff bedeutet oder für den Fall, daß $R^2$ und $R^3$ nicht Wasserstoff bedeuten, Wasserstoff oder ein Rest der Formel IV oder IV′ ist,

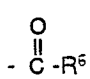 (IV)

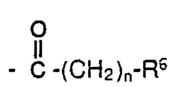 (IV′)

in der

n und $R^6$ die oben angegebenen Bedeutungen haben

und

$R^5$ Wasserstoff oder ein Rest der Formel -$(CH_2)_n$-$R^6$ ist,

wobei

n und $R^6$ die oben angegebenen Bedeutungen haben

und worin

$R^7$ Wasserstoff oder - sofern $R^2$, $R^3$ und $R^4$ nicht gleichzeitig Wasserstoff bedeuten - einen Rest der Formel IV oder IV′ - wie oben definiert -bildet

und in der

$R^{1'}$ die gleiche Bedeutung wie $R^1$ hat, wobei die beiden Reste $R^1$ und $R^{1'}$ sowohl gleich als auch - bis auf die Reste $R^4$ und $R^5$ und für den Fall, daß $R^2$ und $R^3$ zusammen einen Rest der Formel VI bilden - verschieden substituiert sein können, mit der Ausnahme, daß wenn $R^1$ ein Rest der Formel II ist, in der $R^2$ und $R^3$ ein Rest der Formel VIII oder in der $R^2$ und $R^3$ zusammen einen Rest der Formel $>C=X$ bilden, für $R^{1'}$ die Reste $R^2$ und $R^3$ im Rest der Formel II oder III nicht gleichzeitig Wasserstoff sind, ausgenommen Elaiophylin selbst, Elaiophylin mit hydriertem Macrodiolidring, sowie Verbindungen der Formel I, worin der Macrcdiolidring hydriert oder nicht hydriert ist und in der $R^1$ und $R^{1'}$ gleich oder verschieden sind und einen Rest der Formel II und/oder III darstellen, worin $R^2$ und $R^3$ Wasserstoff oder Acetyl und $R^4$ und $R^5$ oder $R^7$ Wasserstoff bedeutet sowie ebenfalls ausgenommen die Verbindungen der Formel I, in der der Macrodiolidring nicht hydriert ist und worin $R^1$ und $R^{1'}$ gleichzeitig einen Rest der Formel II darstellen, worin $R^2$, $R^3$ und $R^4$ Wasserstoff bedeuten und worin

$R^5$ $C_1$-, $C_2$-, $C_4$-$C_8$, $C_{10}$- oder $C_{12}$-Alkyl, Phenyl-$C_1$-$C_3$-alkyl, para-Methoxy-phenyl-methyl, Cyclohexylmethyl, $C_4$-Alkenyl oder $C_3$-Alkinyl

bedeutet.

2. Elaiophylinderivate der Formel I gemäß Anspruch 1, wobei die C-C-Doppelbindungen in dem Macrodiolidring der Verbindung der Formel I auch hydriert sein können und in der $R^1$ ein Rest der Formel II oder III - wie in Anspruch 1 angegeben - ist, worin

$R^2$ und $R^3$ gleich oder verschieden sind und Wasserstoff oder einen Rest der Formel IV oder IV' - wie in Anspruch 1 angegeben - bedeutet, in der

n 1 bis 3 und

$R^6$ $C_1$-$C_5$-Alkyl, $C_2$-$C_5$-Alkenyl, $C_2$-$C_6$-Alkinyl, $C_3$-$C_6$-Cycloalkyl, Phenyl oder Furyl oder Thienyl bedeutet, wobei die Phenyl-, Furyl- und Thienylreste gegebenenfalls halogen-, nitro-, cyano-, hydroxy-, $C_1$-$C_4$-alkyl- oder $C_1$-$C_4$-alkoxy-substituiert sind

und wobei für den Fall, daß $R^3$ Wasserstoff ist, $R^2$ auch Wasserstoff ist,

oder worin

$R^2$ und $R^3$ gleich sind und MEM, MOM, SEM, Trimethylsilyl, Triethylsilyl, Dimethylphenylsilyl, Dimethylcyclohexylsilyl oder Dimethyltertiärbutylsilyl bedeutet

oder

$R^2$ und $R^3$ Sulfonsäureester der Formel $SO_2R^{10}$ bedeuten,

in der

$R^{10}$ $C_1$-$C_4$-Alkyl, Phenyl oder p-Toloyl bedeutet

oder

$R^2$ und $R^3$ einen Rest der Formel V oder V' - wie in Anspruch 1 angegeben - darstellen,

in der

n 1,

X Sauerstoff,

$R^{12}$ $C_1$-$C_4$-Alkyl, $C_3$-$C_6$-Cycloalkyl oder Aryl bedeutet, wobei der Arylrest gegebenenfalls halogen-, nitro-, cyano- oder $C_1$-$C_4$-alkoxysubstituiert ist und

Z Sauerstoff oder -N-H bedeutet

oder worin

$R^2$ und $R^3$ zusammen einen Rest der Formel VI - wie in Anspruch 1 angegeben - darstellen

in der

$R^8$ und $R^9$ gleich oder verschieden sind und Wasserstoff oder unverzweigtes $C_1$-$C_4$-Alykl bedeuten oder wobei

$R^8$ und $R^9$ eine Alkylenkette darstellt, die zusammen mit dem sie tragenden C-Atom einen 5- oder 6-gliedrigen Ring bildet

oder worin

$R^2$ und $R^3$ für den Fall, daß $R^1$ ein Rest der Formel II - wie in Anspruch 1 angegeben - ist, gleich sind und einen Rest der Formel VIII - wie in Anspruch 1 angegeben - darstellen,

in der

X Sauerstoff bedeutet

oder

$R^2$ und $R^3$ für den Fall, daß $R^1$ ein Rest der Formel II - wie in Anspruch 1 angegeben - ist, zusammen einen Rest der Formel $>C=X$ darstellen,

worin

X Sauerstoff bedeutet

und worin

$R^4$ sofern $R^2$ und $R^3$ Wasserstoff sind, Wasserstoff bedeutet oder für den Fall, daß $R^2$ und $R^3$ nicht

Wasserstoff bedeuten, Wasserstoff oder ein Rest der Formel IV oder IV′ - wie in Anspruch 1 angegeben - ist,
in der
n 1 bis 3 ist und $R^6$ die vorstehend (in Anspruch 2) angegebenen Bedeutungen hat
und
$R^5$ Wasserstoff oder ein Rest der Formel $-(CH_2)_n-R^6$ ist,
wobei
n 1 bis 3 ist und $R^6$ die vorstehend (in Anspruch 2) angegebenen Bedeutungen hat
und worin
$R^7$ Wasserstoff oder - sofern $R^2$, $R^3$ und $R^4$ nicht gleichzeitig Wasserstoff bedeuten - einen Rest der Formel IV oder IV′ - wie in Anspruch 1 angegeben und vorstehend (in Anspruch 2) definiert - bildet
und in der
$R^{1'}$ die gleiche Bedeutung wie $R^1$ hat, wobei die beiden Reste $R^1$ und $R^{1'}$ sowohl gleich als auch - bis auf die Reste $R^4$ und $R^5$ und für den Fall, daß $R^2$ und $R^3$ zusammen einen Rest der Formel VI bilden - verschieden substituiert sein können, mit der Ausnahme, daß wenn $R^1$ ein Rest der Formel II - wie in Anspruch 1 angegeben - ist, in der $R^2$ und $R^3$ ein Rest der Formel VIII - wie in Anspruch 1 angegeben - oder in der $R^2$ und $R^3$ zusammen einen Rest der Formel >C=X bilden, für $R^{1'}$ die Reste $R^2$ und $R^3$ im Rest der Formel II oder III - wie in Anspruch 1 angegeben -nicht gleichzeitig Wasserstoff sind, ausgenommen Elaiophylin selbst, Elaiophylin mit hydriertem Macrodiolidring, sowie Verbindungen der Formel I, worin der Macrodiolidring hydriert oder nicht hydriert ist und in der $R^1$ und $R^{1'}$ gleich oder verschieden sind und einen Rest der Formel II und/oder III darstellen, worin $R^2$ und $R^3$ Wasserstoff oder Acetyl und $R^4$ und $R^5$ oder $R^7$ Wasserstoff bedeutet sowie ebenfalls ausgenommen die Verbindungen der Formel I, in der der Macrodiolidring nicht hydriert ist und worin $R^1$ und $R^{1'}$ gleichzeitig einen Rest der Formel II darstellen, worin $R^2$, $R^3$ und $R^4$ Wasserstoff bedeuten und worin
$R^5$ $C_1$-, $C_2$-, $C_4$-$C_5$-Alkyl, Phenyl-$C_1$-$C_3$-Alkyl, para-Methoxy-phenyl-methyl, Cyclohexylmethyl, $C_4$-Alkenyl oder $C_3$-Alkinyl
bedeutet.

3. Elaiophylinderivate der Formel I gemäß Anspruch 1, wobei die C-C-Doppelbindungen in dem Macrodiolidring der Verbindung der Formel I auch hydriert sein können und in der $R^1$ ein Rest der Formel II oder III - wie in Anspruch 1 angegeben - ist,
worin
$R^2$ und $R^3$ gleich oder verschieden sind und Wasserstoff oder einen Rest der Formel IV oder IV′ - wie in Anspruch 1 angegeben -
bedeuten, in der
n 1
$R^6$ $C_1$-$C_5$-Alkyl, Cyclohexyl, Phenyl, Furyl oder Thienyl bedeutet, wobei die Phenyl-, Furyl-, und Thienylreste gegebenenfalls mit Fluor, Chlor oder Brom substituiert sind
und wobei für den Fall, daß $R^3$ Wasserstoff ist, $R^2$ auch Wasserstoff ist,
oder worin
$R^2$ und $R^3$ gleich sind und MEM, MOM, SEM, Trimethylsilyl, Triethylsilyl, Dimethylphenylsilyl, Dimethylcyclohexylsilyl oder Dimethyltertiärbutylsilyl bedeutet
oder
$R^2$ und $R^3$ Sulfonsäureester der Formel $SO_2R^{10}$ bedeuten, in der
$R^{10}$ Methyl, Phenyl oder p-Toloyl bedeutet
oder
$R^2$ und $R^3$ einen Rest der Formel V oder V′ - wie in Anspruch 1 angegeben - darstellen,
in der
n 1
X Sauerstoff
$R^{12}$ Cyclohexyl oder Aryl bedeutet, wobei der Arylrest gegebenenfalls halogen-, nitro-, cyano- oder $C_1$-$C_4$-alkoxysubstituiert ist und
Z Sauerstoff oder -N-H bedeutet,
oder worin
$R^2$ und $R^3$ zusammen einen Rest der Formel VI - wie in Anspruch 1 angegeben - darstellen
in der
$R^8$ und $R^9$ gleich oder verschieden sind und Wasserstoff oder unverzweigtes $C_1$-$C_4$-Alykl bedeuten oder wobei
$R^8$ und $R^9$ eine Alkylenkette darstellt, die zusammen mit dem sie tragenden C-Atom einen 5- oder 6-

gliedrigen Ring bildet
oder worin
$R^2$ und $R^3$ für den Fall, daß $R^1$ ein Rest der Formel II
- wie in Anspruch 1 angegeben - ist, gleich sind und einen Rest der Formel VIII - wie in Anspruch 1 angegeben - darstellen,
in der
X Sauerstoff bedeutet
oder
$R^2$ und $R^3$ für den Fall, daß $R^1$ ein Rest der Formel II - wie in Anspruch 1 angegeben - ist, zusammen einen Rest der Formel $\geq C=X$ darstellen,
worin
X Sauerstoff bedeutet
und worin
$R^4$ sofern $R^2$ und $R^3$ Wasserstoff sind, Wasserstoff bedeutet oder für den Fall, daß $R^2$ und $R^3$ nicht Wasserstoff bedeuten, Wasserstoff oder ein Rest der Formel IV oder IV′ - wie in Anspruch 1 angegeben - ist,
in der
n 1 ist und $R^6$ die vorstehend (in Anspruch 3) angegebenen Bedeutungen hat
und
$R^5$ Wasserstoff oder ein Rest der Formel $-(CH_2)_n-R^6$ ist,
wobei
n 1 ist und $R^6$ die vorstehend (in Anspruch 3) angegebenen Bedeutungen hat
und worin
$R^7$ Wasserstoff oder - sofern $R^2$, $R^3$ und $R^4$ nicht gleichzeitig Wasserstoff bedeuten - einen Rest der Formel IV oder IV′ - wie in Anspruch 1 angegeben und vorstehend (in Anspruch 3) definiert - bildet
und in der
$R^{1'}$ die gleiche Bedeutung wie $R^1$ hat, wobei die beiden Reste $R^1$ und $R^{1'}$ sowohl gleich als auch
- bis auf die Reste $R^4$ und $R^5$ und für den Fall, daß $R^2$ und $R^3$ zusammen einen Rest der Formel VI bilden - verschieden substituiert sein können, mit der Ausnahme, daß wenn $R^1$ ein Rest der Formel II
- wie in Anspruch 1 angegeben - ist, in der $R^2$ und $R^3$ ein Rest der Formel VIII - wie in Anspruch 1 angegeben - oder in der $R^2$ und $R^3$ zusammen einen Rest der Formel $\geq C=X$ bilden, für $R^{1'}$ die Reste $R^2$ und $R^3$ im Rest der Formel II oder III - wie in Anspruch 1 angegeben - nicht gleichzeitig Wasserstoff sind, ausgenommen Elaiophylin selbst, Elaiophylin mit hydriertem Macrodiolidring, sowie Verbindungen der Formel I, worin der Macrodiolidring hydriert oder nicht hydriert ist und in der $R^1$ und $R^{1'}$ gleich oder verschieden sind und einen Rest der Formel II und/oder III darstellen, worin $R^2$ und $R^3$ Wasserstoff oder Acetyl und $R^4$ und $R^5$ oder $R^7$ Wasserstoff bedeutet sowie ebenfalls ausgenommen die Verbindungen der Formel I, in der der Macrodiolidring nicht hydriert ist und worin $R^1$ und $R^{1'}$ gleichzeitig einen Rest der Formel II darstellen, worin $R^2$, $R^3$ und $R^4$ Wasserstoff bedeuten und worin
$R^5$ $C_1$-$C_2$-, $C_4$-$C_5$-Alkyl, Phenylmethyl oder Cyclohexylmethyl, bedeutet.

4. Elaiophilinderivate der Formel I, gemäß Anspruch 1, jedoch einschließlich Elaiophylin mit hydriertem Macrodiolidring, sowie Verbindungen der Formel I, worin der Macrodiolidring hydriert oder nicht hydriert ist und in der $R^1$ und $R^{1'}$ gleich oder verschieden sind und einen Rest der Formel II und/oder III darstellen, worin $R^2$ und $R^3$ Wasserstoff oder Acetyl und $R^4$ und $R^5$ oder $R^7$ Wasserstoff bedeutet sowie ebenfalls eingeschlossen die Verbindungen der Formel I, in der der Macrodiolidring nicht hydriert ist und worin $R^1$ und $R^{1'}$ gleichzeitig einen Rest der Formel II darstellen, worin $R^2$, $R^3$ und $R^4$ Wasserstoff bedeuten und worin
$R^5$ $C_1$-, $C_2$-, $C_4$-$C_8$, $C_{10}$- oder $C_{12}$-Alkyl, Phenyl-$C_1$-$C_3$-alkyl, para-Methoxy-phenyl-methyl, Cyclohexylmethyl, $C_4$-Alkenyl oder $C_3$-Alkinyl
bedeutet,
zur Anwendung als Anthelminthikum.

5. Elaiophylinderivate der Formel I gemäß Anspruch 2, jedoch einschließlich Elaiophylin mit hydriertem Macrodiolidring, sowie Verbindungen der Formel I, worin der Macrdiolidring hydriert oder nicht hydriert ist und in der $R^1$ und $R^{1'}$ gleich oder verschieden sind und einen Rest der Formel II und/oder III darstellen, worin $R^2$ und $R^3$ Wasserstoff oder Acetyl und $R^4$ und $R^5$ oder $R^7$ Wasserstoff bedeutet sowie ebenfalls eingeschlossen die Verbindungen der Formel I, in der der Macrodiolidring nicht hydriert ist und worin $R^1$ und $R^{1'}$ gleichzeitig einen Rest der Formel II darstellen, worin $R^2$, $R^3$ und $R^4$ Wasserstoff bedeuten und worin
$R^5$ $C_1$-, $C_2$-, $C_4$-$C_5$-Alkyl, Phenyl-$C_1$-$C_3$-Alkyl, para-Methoxy-phenyl-methyl, Cyclohexylmethyl, $C_4$-Alkenyl

oder $C_3$-Alkinyl
bedeutet,
zur Anwendung als Anthelminthikum.

6. Elaiophylinderivate der Formel I gemäß Anspruch 3, jedoch einschließlich Elaiophylin mit hydriertem Macrodiolidring, sowie Verbindungen der Formel I, worin der Macrodiolidring hydriert oder nicht hydriert ist und in der $R^1$ und $R^{1'}$ gleich oder verschieden sind und einen Rest der Formel II und/oder III darstellen, worin $R^2$ und $R^3$ Wasserstoff oder Acetyl und $R^4$ und $R^5$ oder $R^7$ Wasserstoff bedeutet sowie ebenfalls eingeschlossen die Verbindungen der Formel I, in der der Macrodiolidring nicht hydriert ist und worin $R^1$ und $R^{1'}$ gleichzeitig einen Rest der Formel II darstellen, worin $R^2$, $R^3$ und $R^4$ Wasserstoff bedeuten und worin
$R^5$ $C_1$-, $C_2$-, $C_4$-$C_5$-Alkyl, Phenylmethyl oder Cyclohexylmethyl, bedeutet,
zur Anwendung als Anthelminthikum.

7. Verfahren zur Herstellung von Elaiophylinderivaten der Formel I gemäß Anspruch 1, dadurch gekennzeichnet, daß man

a) Elaiophylin (Formel I: $R^1$ = $R^{l}$ = II, $R^2$ = $R^3$ = $R^4$ = $R^5$ = H) oder eine Verbindung der Formel I, in der $R^1$ und $R^{1'}$ gleich oder verschieden sind und einen Rest der Formel II und/oder III darstellen, worin $R^2$, $R^3$ und $R^4$ die in Anspruch 1 angegeben Bedeutungen haben und $R^5$ oder $R^7$ bis auf Wasserstoff die in Anspruch 1 angegebenen Bedeutungen haben, umsetzt mit einer Verbindung der Formel IX oder IX′

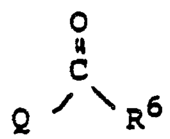 (IX) 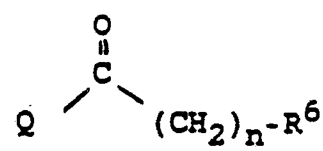 (IX')

in der n und $R^6$ die in Anspruch 1 zu Formal IV oder IV′ angegebenen Bedeutungen haben und Q Chlorid, Bromid, ein Imidazolid oder ein Säureanhydrid bedeutet, wobei man Verbindungen der Formel I erhält, in der $R^1$ und $R^{1'}$ gleich oder verschieden sind und einen Rest der Formel II und/oder III darstellen, in der $R^2$ und $R^3$ gleich oder verschieden sind und einen Rest der Formel IV oder IV′, wie in Anspruch 1 angegeben, darstellen, und in der $R^4$ und $R^5$ oder $R^7$ gegenüber der Ausgangsverbindung unverändert bleiben oder daß man

b) ein Elaiophylin-Derivat der Formel I in der $R^1$ und $R^{1'}$ gleich oder verschieden sind und einen Rest der Formel II und/oder III darstellen, worin $R^2$, $R^3$ und $R^4$ die in Anspruch 1 angegebenen Bedeutungen haben und $R^5$ oder $R^7$ bis auf Wasserstoff die in Anspruch 1 angegebenen Bedeutungen haben, umsetzt mit einer Verbindung, ausgewählt aus: $C_1$-$C_4$-Alkyl-, Benzyl-, Allylchlorid, -bromid, -jodid oder MEM-, MOM-, SEM-chlorid, Triemethylsilyl-, Triethylsilyl-, Dimethylphenylsilyl-, Dimethylcyclohexylsilyl-, Dimethyltertiärbutylsilyl-chlorid, Sulfonsäurehalogeniden der Formel Y-$SO_2R^{10}$, wobei Y Chlor, Brom oder Jod bedeutet und $R^{10}$ die in Anspruch 1 angegebenen Bedeutungen hat, Isocyanaten der Formel O = C = N-$R^{12}$ oder
O = C = N-$(CH_2)_n$-$R^{12}$, Isothiocyanaten der Formel S = C = N-$R^{12}$ oder
S = C = N-$(CH_2)_n$-$R^{12}$, Carbaminsäurehalogeniden der Formel Y-CO-N-$(R^{12})_2$ oder
Y-CO-N-$[(CH_2)_n$-$R^{12}]_2$, Thiocarbaminsäurehalogeniden der Formel Y-CS-N-$(R^{12})_2$ oder
Y-CS-N-$[(CH_2)_n$-$R^{12}]_2$,
wobei n und $R^{12}$ die in Anspruch 1 zu Formel V oder V′ angegebenen Bedeutungen haben und Y Chlor oder Brom bedeutet,
wobei man Verbindungen der Formel I erhält, in der $R^1$ und $R^{1'}$ gleich oder verschieden sind und einen Rest der Formel II und/oder III darstellen, in der $R^2$ und $R^3$ gleich sind und einen
$C_1$-$C_4$-Alkyl-, Benzyl-, Allyl-, MEM-, MOM-, SEM-, Trimethylsilyl-, Triethylsilyl-, Dimethylphenylsilyl-, Dimethylcyclohexylsilyl- oder Dimethyltertiärbutylsilyl-Rest darstellen oder einen Sulfonsäureester der Formel $SO_2R^{10}$ oder einen Rest der Formel V oder V′ bedeuten, wobei $R^{10}$ und die Formeln V und V′ die in Anspruch 1 angegebenen Bedeutungen haben und in der $R^4$ und $R^5$ oder $R^7$ gegenüber der Ausgangsverbindung unverändert sind, oder daß man

c) Elaiophylin (Formel I: $R^1$ = $R^{1'}$ = II, $R^2$ = $R^3$ = $R^4$ = $R^5$ = H) umsetzt mit einem Aldehyd/Keton der Formel X

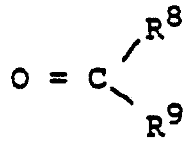 (X)

oder mit einem Ketal der Formel XI

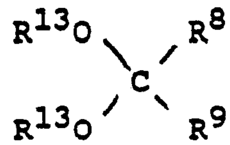 (XI)

worin $R^{13}$ Methyl oder Ethyl bedeutet und $R^8$ und $R^9$ die in Anspruch 1 angegebenen Bedeutungen haben, wobei man Verbindungen der Formel I erhält, in der $R^1$ und $R^{1'}$ gleich sind und einen Rest der Formel II darstellen, in der $R^2$ und $R^3$ zusammen einen Rest der Formel VI, wie in Anspruch 1 angegeben, darstellen oder daß man

d) Elaiophylin (Formel I: $R^1 = R^{1'} = II$, $R^2 = R^3 = R^4 = R^5 = H$) oder eine Verbindung der Formel I, in der $R^1$ und $R^{1'}$ gleich oder verschieden sind und einen Rest der Formel II darstellen, worin $R^2$, $R^3$ und $R^4$ die in Anspruch 1 angegebenen Bedeutungen haben und $R^5$ bis auf Wasserstoff die in Anspruch 1 angegebenen Bedeutungen hat, gegebenenfalls in Gegenwart einer Base umsetzt mit Carbonyldiimidazol oder Thiocarbonyldiimidazol, wobei man Verbindungen der Formel I erhält, in der $R^1$ und $R^{1'}$ gleich oder verschieden sind und einen Rest der Formel II darstellen, in der $R^2$ und $R^3$ gleich sind und einen Rest der Formel VIII, wie in Anspruch 1 angegeben, darstellen oder in der $R^2$ und $R^3$ zusammen einen Rest der Formel $>C=X$, wie in Anspruch 1 angegeben, darstellen und in der $R^4$ und $R^5$ gegenüber der Ausgangsverbindung unverändert sind, oder daß man

e) eine Verbindung der Formel I, in der $R^1$ und $R^{1'}$ gleich oder verschieden sind und einen Rest der Formel II und/oder III darstellen, in der $R^2$ und $R^3$ bis auf Wasserstoff die in Anspruch 1 angegebenen Bedeutungen haben und in der $R^4$ Wasserstoff bedeutet und $R^5$ oder $R^7$ bis auf Wasserstoff die in Anspruch 1 angegebenen Bedeutungen hat, gemäß Verfahrensvariante a) mit einer Verbindung der Formel IX oder IX', wie unter a) definiert, umsetzt, wobei man Verbindungen der Formel I erhält, in der $R^1$ und $R^{1'}$ gleich oder verschieden sind und einen Rest der Formel II und/oder III darstellen, in der $R^2$ und $R^3$ bis auf Wasserstoff die in Anspruch 1 angegebenen Bedeutungen haben und $R^4$ ein Rest der Formel IV oder IV', wie in Anspruch 1 angegeben, bedeutet, wobei $R^4$ in $R^{1'}$ identisch ist mit $R^4$ in $R^1$ und in der $R^5$ oder $R^7$ gegenüber der Ausgangsverbindung unverändert ist, oder daß man

f) Elaiophylin oder eine Verbindung der Formel I, in der $R^1$ und $R^{1'}$ gleich oder verschieden sind und einen Rest der Formel II darstellen, in der $R^2$, $R^3$ und $R^4$ die in Anspruch 1 angegebenen Bedeutungen haben und in der $R^5$ Wasserstoff bedeutet, bevorzugt in Gegenwart einer Lewis-Säure mit einem Alkohol der Formel $HO-(CH_2)_n-R^6$
in der n und $R^6$ die in Anspruch 1 angegebenen Bedeutungen haben, umsetzt, wobei man Verbindungen der Formel I erhält, in der $R^1$ und $R^{1'}$ gleich oder verschieden sind und einen Rest der Formel II darstellen, in der $R^2$ und $R^3$ und $R^4$ die in Anspruch 1 angegebenen Bedeutungen haben und $R^5$ in den Resten $R^1$ und $R^{1'}$ identisch ist und einen Rest der Formel $-(CH_2)_n-R^6$ oder $-R^6$, wie in Anspruch 1 angegeben, darstellt, oder daß man

g) Elaiophylin (Formel I: $R^1 = R^{1'} = II$, $R^2 = R^3 = R^4 = R^5 = H$) oder eine Verbindung der Formel I, in der $R^1$ und $R^{1'}$ gleich oder verschieden sind und einen Rest der Formel II darstellen, worin $R^2$, $R^3$ und $R^4$ die in Anspruch 1 angegebenen Bedeutungen haben und $R^5$ Wasserstoff ist, wobei für $R^2$ und $R^3$ die Substituenten der Formel VIII sowie die Carbonyl- und Thiocarbonylreste ausgeschlossen sind, reduziert, wobei man eine Verbindung der Formel I erhält, in der $R^1$ und/oder $R^{1'}$ ein Rest der Formel III ist, worin $R^2$, $R^3$ und $R^4$ die in Anspruch 1 angegebenen Bedeutungen haben und $R^7$ Wasserstoff bedeutet, oder daß man

h) eine Verbindung der Formel I, in der $R^1$ und/oder $R^{1'}$ ein Rest der Formel III ist, worin $R^2$, $R^3$ und $R^4$ die in Anspruch 1 angegebenen Bedeutungen haben und $R^7$ Wasserstoff bedeutet, gemäß Verfahrensvariante a) mit einer Verbindung der Formel IX oder IX', wie in Verfahrensvariante a) definiert, zu einer Verbindung der Formel I umsetzt, in der $R^1$ und/oder $R^{1'}$ ein Rest der Formel III ist, worin $R^2$, $R^3$ und $R^4$ die

in Anspruch 1 angegebenen Bedeutungen haben oder, sofern vor der Umsetzung $R^2$ und/oder $R^3$ und/oder $R^4$ Wasserstoff bedeuteten, $R^2$ und/oder $R^3$ und/oder $R^4$ nach der Umsetzung die Bedeutung von $R^7$ haben und in der $R^7$ ein Rest der Formel IV oder IV', wie in Anspruch 1 angegeben, ist, oder daß man

i) eine Verbindung der Formel I, in der $R^1$ und/oder $R^{1'}$ ein Rest der Formel II und/oder III ist, worin $R^2$, $R^3$, $R^4$ und $R^5$ oder $R^7$ die in Anspruch 1 angegebenen Bedeutungen haben, hydriert, wobei man eine hydrierte Verbindung der Formel I erhält, in der $R^1$ und/oder $R^{1'}$ ein Rest der Formel II und/oder III ist, worin $R^2$, $R^3$, $R^4$ und $R^5$ oder $R^7$ die in Anspruch 1 angegebenen Bedeutungen haben.

8. Verbindungen nach einem der Ansprüche 1 bis 3 zur Anwendung als Arzneimittel.

9. Arzneimittel, enthaltend eine Verbindung der Formel I gemäß Anspruch 1.

10. Verfahren zur Herstellung von Arzneimitteln, welche anthelminthisch wirken, dadurch gekennzeichnet, daß man in das Arzneimittel eine Verbindung der Formel I gemäß Anspruch 4 einverleibt.